# FASCICULE DE BREVET EUROPEEN

(11) **EP 1 828 256 B1**
(45) Date de publication et mention de la délivrance du brevet: **11.02.2015**
(21) Numéro de dépôt: 05824383.3
(22) Date de dépôt: 08.12.2005
(51) Int. Cl.: C08F 2/32, C08F 220/54

(54) **NOUVEAU LATEX INVERSE CONCENTRE, PROCEDE POUR SA PREPARATION, ET UTILISATION DANS L'INDUSTRIE**
NEUER KONZENTRIERTER POSITIVER LATEX, VERFAHREN ZU SEINER HERSTELLUNG UND INDUSTRIELLE VERWENDUNG
NOVEL CONCENTRATED POSITIVE LATEX, METHOD FOR THE PREPARATION THEREOF, AND USE THEREOF IN INDUSTRY

(30) Priorité: 16.12.2004 FR 0453017
(43) Date de publication de la demande: 05.09.2007
(73) Titulaire: SOCIETE D'EXPLOITATION DE PRODUITS POUR LES INDUSTRIES CHIMIQUES-SEPPIC, 75321 Paris Cedex 07 (FR)
(72) Inventeur: BRAUN, Olivier, F-81100 Castres (FR); MALLO, Paul, F-78290 Croissy-sur-Seine (FR)
(74) Mandataire: Conan, Philippe Claude
(86) Numéro de dépôt international: PCT/FR2005/051058
(87) Numéro de publication internationale: WO 2006/064151

(56) Documents cités:
- EP-A- 0 896 966
- CHEMICAL ABSTRACTS, vol. 115, no. 16, 21 octobre 1991 (1991-10-21), Columbus, Ohio, US; abstract no.: 160037,"MANUFACTURE OF HYDROPHILIC RESIN PARTICLES" page 16 XP000388707 & JP 03 903802 A (SANYO CHEM. IND.) 18 avril 1991 (1991-04-18)

## Description

La présente demande concerne des latex inverse eau dans huile, leur procédé de préparation et leur application en tant qu'épaississant et/ou émulsionnant dans les produits industriels, les produits de soins de la peau et des cheveux ou pour la fabrication de préparations cosmétiques, dermopharmaceutiques ou pharmaceutiques.

Les latex inverses de polymères de l'acide l'acide 2-méthyl 2-[(1-oxo 2-propènyl) amino] 1-propanesulfonique (dénommé aussi acide 2-acrylamido 2-méthyl propanesulfonique, ATBS ou AMPS) partiellement ou totalement salifié ainsi que leur utilisation en cosmétique et/ou pharmacie ont fait l'objet de nombreuses demandes de brevet. Cependant la présence de quantités importantes d'eau et d'huile représente un inconvénient non négligeable en termes de volume, de coût et parfois de risques accrus et/ou d'effets toxiques.

Des solutions ont donc été développées pour augmenter la concentration en polymères dans les latex finaux par exemple en soumettant le milieu réactionnel, en fin de polymérisation, à une étape de distillation sous vide pour enlever une partie plus ou moins importante d'eau et d'huile. Cette distillation est cependant délicate à mettre en oeuvre car elle induit souvent une déstabilisation du latex inverse qu'il faut contrer par l'addition préalable d'agents stabilisants. Les demandes de brevet européen EP 0 161 038 et EP 0 126 528 ainsi que la demande de brevet britannique GB 1 482 515 divulguent une telle utilisation de polymères stabilisants.

L'inconvénient de ces produits est qu'ils contiennent des alcools ou des glycols pouvant induire des problèmes environnementaux. De plus il se produit parfois une prise en masse du milieu réactionnel lors de l'étape de distillation sans que ce phénomène n'ait jamais vraiment été expliqué, mais dont la conséquence certaine est la destruction du lot de latex inverse et un nettoyage pénible et coûteux du réacteur. Enfin, même quand la distillation se déroule correctement, les latex inverses obtenus s'inversent souvent difficilement, ils ont une viscosité élevée et présentent parfois en leur sein des micro-gels. Ces inconvénients interdisent donc leur utilisation dans la fabrication de formulations cosmétiques et/ou d'impression textile.

C'est pourquoi la demanderesse s'est attachée à mettre au point des latex inverses concentrés, c'est à dire comprenant au moins 50 % en poids de polymère et moins de 5 % en poids d'eau dépourvus de tels inconvénients et ayant une tenue aux électrolytes améliorée.

Selon un premier aspect, l'invention a pour objet une composition sous forme d'un latex inverse comprenant :
a) de 50 % en poids à 80 % d'au moins un polymère organique (P) linéaire, branché ou réticulé,
b) de 5 % en poids à 10 % d'un système émulsionnant (S₁) de type eau dans huile (E/H),
c) de 5 % en poids à 45 % en poids d'au moins une huile, et
d)de0%à5%d'eau,
et caractérisée en ce que de 0,01 % à 10 % en proportions molaires des motifs monomériques que ledit polymère P comporte est au moins un monomère neutre de formule (I) :

C(R₁)(R₃)=C(R₂)-C(=0)-0-(CH₂-CH₂-O)ₙ-R₄ (I)

dans laquelle les radicaux R₁, R₂ et R₃, identiques ou différents représentent indépendamment les uns des autres un atome d'hydrogène ou un radical alkyle linéaire ou ramifié comportant de 1 à 4 atomes de carbone, le radical R₄ représente un radical aliphatique linéaire ou ramifié, saturé ou insaturé, comportant de 6 à 30 atomes de carbone et n représente un nombre compris entre 1 et 50.

Par radical alkyle linéaire ou ramifié comportant de 1 à 4 atomes de carbone, on désigne pour les radicaux R₁, R₂ et R₃, les radicaux méthyle, éthyle, propyle, isopropyle, butyle, isobutyle, sec- butyle ou tert-butyle.

Par radical aliphatique linéaire ou ramifié, saturé ou insaturé, comportant de 6 à 30 atomes de carbone, on désigne pour le radical R₄, plus particulièrement les radicaux linéaires saturés tels que, par exemple, les radicaux, hexyle, octyle, nonyle, décyle, undécyle, dodécyle, tétradécyle, hexadécyle, octadécyle, eicosyle ou docosyle ou tétracosyle ou hexacosyle ou octacosyle ou triacontyle.

L'invention a plus particulièrement pour objet une composition telle que définie à la revendication 1, dans laquelle de 0,05 % à 5 % en proportions molaires, et de préférence de 0,1 % à 1% en proportions molaires, des motifs monomériques que ledit polymère P comporte est au moins un monomère neutre de formule (I)

La composition telle que définie précédemment contient, soit un seul polymère (P), soit un mélange de polymères (P) différents.

Selon un premier aspect particulier de la présente invention, le polymère (P) est :
- soit un copolymère dans lequel chacun des monomères différents du composé de formule (I), est choisi indépendamment l'un de l'autre ou bien parmi ceux possédant une fonction acide fort partiellement ou totalement salifiée ou bien parmi ceux possédant une fonction acide faible partiellement ou totalement salifié ou bien parmi les monomères neutres ou bien parmi les monomères cationiques.

Dans la composition telle que définie ci-dessus, le système émulsionnant (S₁) de type eau dans huile (E/H) est constitué soit d'un seul tensioactif soit d'un mélange de tensioactifs à condition que ledit mélange ait une valeur de HLB suffisamment faible pour induire des émulsions eau dans huile. Comme agent émulsionnant de type eau - dans huile, il y a par exemple les esters de sorbitan, comme l'oléate de sorbitan, comme celui commercialisé par la société SEPPIC sous le nom MONTANE™ 80, l'isostéarate de sorbitan, comme celui commercialisé par la société SEPPIC sous le nom MONTANE™ 70 ou le sesquioléate de sorbitan comme celui commercialisé par la société SEPPIC sous le nom MONTANE™ 83. Il y aussi certains esters de sorbitan polyéthoxylés, par exemple le monoléate de sorbitan pentaéthoxylé comme celui commercialisé par la société SEPPIC sous le nom MONTANOX™ 81 ou l'isostéarate de sorbitan pentaéthoxylé comme celui commercialisé sous le nom MONTANOX™ 71 par la société SEPPIC. Il y a encore l'alcool oléocétylique diéthoxylé comme celui commercialisé sous le nom SIMULSOL™ OC 72 par la société SEPPIC, les polyesters de poids moléculaire compris entre 1000 et 3000, produits de la condensation entre un acide poly(isobutènyl) succinique ou son anhydride et tels que l'HYPERMER™ 2296 commercialisé par la société UNIQEMA ou enfin les copolymères blocks de poids moléculaire compris entre 2500 et 3500, comme l'HYPERMER™ B246 commercialisé par la société UNIQEMA ou le SIMALINE™ IE 200 commercialisé par la société SEPPIC.

Par polymère branché, on désigne pour (P), un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne pour (P), un polymère non linéaire se présentant à l'état de réseau tridimensionnel insoluble dans l'eau, mais gonflable à l'eau et conduisant donc à l'obtention d'un gel chimique.

La composition selon l'invention peut comporter des polymères linéaires, des polymères réticulés et/ou des polymères branchés.

Lorsque le polymère (P) est réticulé, il l'est plus particulièrement avec un composé diéthylènique ou polyéthylènique dans la proportion molaire exprimée par rapport aux monomères mis en oeuvre, inférieure ou égale à 0,25 %, et plus particulièrement inférieure ou égale à 0,05 % et tout particulièrement entre 0,005 % et 0,01 %. De préférence, l'agent de réticulation et/ou l'agent de ramification est choisi parmi le diméthacrylate d'éthylèneglycol, le diacrylate de diéthylèneglycol, le diallyloxyacétate de sodium, le diacrylate d'éthylèneglycol, le diallyl urée, le triallylamine, le triméthylol propanetriacrylate, le méthylène-bis-(acrylamide) ou un mélange de ces composés.

La fonction acide fort des monomères en comportant est notamment la fonction acide sulfonique ou la fonction acide phosphonique. Lesdits monomères sont par exemple l'acide styrènesulfonique partiellement ou totalement salifié ou, de préférence, l'acide 2-méthyl 2-[(1-oxo 2-propènyl) amino] 1-propanesulfonique (dénommé aussi acide 2-acrylamido 2-méthyl propanesulfonique) partiellement ou totalement salifiée.

La fonction acide faible des monomères en comportant est notamment la fonction acide carboxylique partiellement salifiée. Lesdits monomères peuvent être par exemple l'acide acrylique, l'acide méthacrylique, l'acide itaconique, l'acide maléique ou l'acide 3-méthyl 3-[(1-oxo 2-propènyl) amino] butanoïque partiellement ou totalement salifié.

Pour les monomères à fonction acide fort ou à fonction acide faible, le terme salifié indique qu'il s'agit de sels de métaux alcalins tels que les sels de sodium ou de potassium, les sels de bases azotées comme le sel d'ammonium, le sel de lysine ou le sel de monoéthanolamine (HO-CH₂-CH₂-NH₄⁺).

Les monomères neutres différents du composé de formule (I), sont notamment choisis parmi l'acrylamide, le méthacrylamide, le diacétoneacrylamide, le diméthylacrylamide, le N-isopropyl acrylamide, le N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], l'acrylate de (2-hydroxy éthyle), l'acrylate de (2,3-dihydroxy propyle), le méthacrylate de (2-hydroxy éthyle), le méthacrylate de (2,3-dihydroxy propyle), un dérivé éthoxylé de poids moléculaire compris entre 400 et 1000, de chacun de ces esters ou le vinyl pyrrolidone.

Les monomères cationiques sont notamment choisis parmi les dérivés d'ammonium quaternaires. Lesdits monomères peuvent être par exemple les sels de 2,N,N,N-tétraméthyl 2-[(1-oxo 2-propènyl) amino] propanammonium, de 2,N,N-triméthyl 2-[(1-oxo 2-propènyl) amino] propanammonium, de N,N,N-triméthyl 2-[(1-oxo 2-propènyl) oxy] éthanammonium, de N,N,N-triméthyl 3-[(1-oxo 2-propènyl) oxy] propanammonium, de N,N,N-triméthyl 2-[(1-oxo 2-propènyl) amino] propanammonium, de diallyl diméthyl ammonium. Par sel on entend plus particulièrement les chlorures, les bromures ou les iodures des dits sels d'ammonium.

L'invention a plus particulièrement pour objet, une composition telle que définie précédemment, pour laquelle dans la formule (I), le radical R₄ représente un radical aliphatique linéaire ou ramifié, saturé ou insaturé, comportant de 8 à 24 atomes de carbone, une composition telle que définie précédemment, pour laquelle dans la formule (I), les radicaux R₁, R₂ et R₃, identiques ou différents représentent indépendamment les uns des autres un atome d'hydrogène ou un radical méthyle et une composition telle que définie précédemment, pour laquelle dans la formule (I), n est un nombre compris entre 1 et 30 à et de préférence un nombre compris entre 1 et 25.

Selon un autre aspect particulier, dans la composition telle que définie précédemment, le monomère est choisi parmi les composés de formule (I') :

CH₂=CH-C(=0)-0-(CH₂-CH₂-O)_{n'}-R'₄ (I')

correspondant à la formule (I) dans laquelle les radicaux R₁, R₂ et R₃, représentent chacun un atome d'hydrogène, le radical R₄ représente un radical aliphatique choisi parmi les radicaux ,octyle, decyle, dodecyle tétradécyle, hexadécyle ou octadécyle, eicosyle, docosylé, tétracosyle et n' représente un nombre compris entre 4 et 25 ; ou bien les composés de formule (I") :

CH₂=C(CH₃)-C(=0)-0-(CH₂-CH₂-O)_{n"}-R"₄ (I")

correspondant à la formule (I) dans laquelle les radicaux R₁ et R₃ représentent chacun un atome d'hydrogène, le radical R₂ représente un groupe méthyle, le radical R₄ représente un radical aliphatique choisi parmi les radicaux octyle, dodécyle, tétradécyle, hexadécyle ou octadécyle, eicosyle, docosyle, tétracosyle et n' représente un nombre compris entre 4 et 25

Le polymère (P) est alors de préférence choisi parmi :
- les copolymères réticulés de l'acide acrylique partiellement salifié sous forme de sel de sodium ou de sel d'ammonium, de acrylamide et de l'acrylate de lauryle tétraéthoxylé ;
- les copolymères réticulés de l'acide 2-méthyl 2-[(1-oxo 2-propènyl) amino] 1-propanesulfonique partiellement salifié sous forme de sel de sodium de l'acrylamide et de l'acrylate de lauryle tétraéthoxylé ;
- les copolymères réticulés de l'acide 2-méthyl 2-[(1-oxo 2-propènyl) amino] 1-propanesulfonique de l'acide acrylique partiellement salifiés sous forme de sel de sodium et de l'acrylate de lauryle tétraéthoxylé ;
- les copolymères réticulés de l'acide 2-méthyl 2-[(1-oxo 2-propènyl) amino] 1-propanesulfonique partiellement salifié sous forme de sel de sodium de l'acrylate de 2-hydroxy éthyle et de l'acrylate de lauryle tétraéthoxylé ;
- les copolymères réticulés de l'acrylamide du N,N,N-triméthyl 3-(1-oxo 2-propènyl) propanammonium et de l'acrylate de lauryle tétraéthoxylé ;
- les copolymères réticulés de l'acide 2-méthyl 2-[(1-oxo 2-propènyl) amino] 1-propanesulfonique partiellement salifié sous forme de sel de sodium et de l'acrylate de lauryle tétraéthoxylé ;
- les copolymères réticulés de l'acide acrylique partiellement salifié sous forme de sel d'ammonium ou de sel de monoéthanolamine et de l'acrylate de lauryle tétraéthoxylé ;
- les copolymères de l'acrylamide, du N,N,N-triméthyl 3-(1-oxo 2-propènyl) propanammonium, de tris(hydroxyméthyl)aminométhyl acrylamide et de l'acrylate de lauryle tétraéthoxylé ;
- les copolymères réticulés de l'acrylamide, de l'acide 2-méthyl 2-[(1-oxo 2-propènyl) amino] 1-propanesulfonique de l'acide acrylique partiellement salifiés sous forme de sel sodium et de l'acrylate de lauryle tétraéthoxylé ; et
- les copolymères de l'acide 2-méthyl 2-[(1-oxo 2-propènyl) amino] 1-propanesulfonique partiellement salifié sous forme de sel de sodium, de l'acrylamide, du
vinyl pyrrolidone et de l'acrylate de lauryle tétraéthoxylé .

Selon un autre mode particulier de la présente invention, la composition telle que définie précédemment, comprend au moins de 60 % en poids et au plus 70 % en poids de polymère (P).

Selon un autre mode particulier de la présente invention, la composition telle que définie précédemment, comprend en outre jusqu'à 5 % de son poids d'un système émulsionnant (S₂) de type huile dans eau (H/E).

Par " agent émulsifiant du type huile dans eau ", on désigne des agents émulsifiants possédant une valeur de HLB suffisamment élevée pour fournir des émulsions huile dans l'eau tels que les esters de sorbitan éthoxylés comme l'oléate de sorbitan polyéthoxylé avec 20 moles d'oxyde d'éthylène , commercialisé par la société SEPPIC sous le nom de MONTANOX™ 80, le laurate de sorbitan polyéthoxylé avec 20 moles d'oxyde d'éthylène, commercialisé par la société SEPPIC sous le nom de MONTANOX™ 20, l'huile de ricin polyéthoxylée avec 40 moles d'oxyde d'éthylène commercialisé sous le nom SIMULSOL™ OL50, l'alcool oléodécylique décaéthoxylé, commercialisé par la société SEPPIC sous le nom SIMULSOL™ OC 710, l'alcool laurique heptaéthoxylé commercialisé sous le nom SIMULSOL™ P7, le nonylphénol décaéthoxylé commercialisé par la société SEPPIC sous le nom NONAROX™-10-30 ou les hexaoléates de sorbitan polyéthoxylés commercialisés par la société SEPPIC sous le nom SIMALINE™-IE 400.

Dans la composition objet de la présente invention, la phase huile est constituée soit par une huile minérale commerciale contenant des hydrocarbures saturés comme les paraffines, les isoparaffines, les cycloparaffines, présentant à température ambiante, une densité entre 0,7 et 0,9 et un point d'ébullition supérieur à environ 250°C, telle que par exemple le MARCOL™52 commercialisés par EXXON CHEMICAL, soit par une huile végétale comme le squalane d'origine végétale, soit une huile de synthèse tel que le polyisobutène hydrogéné ou le polydécène hydrogéné, soit par un mélange de plusieurs de ces huiles. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993). La composition selon l'invention peut également contenir divers additifs tels que des agents complexants, des agents de transfert ou des agents limiteurs de chaîne.

Selon un autre aspect de la présente invention, celle-ci a pour objet un procédé de préparation de la composition telle que définie précédemment, caractérisé en ce que :
a) l'on émulsionne une phase aqueuse (A) contenant les monomères et les éventuels additifs hydrophiles, dans une phase organique (O) contenant, le système tensioactif (S₁), un mélange constitué de l'huile destinée à être présente dans la composition finale et d'une huile volatile et les éventuels additifs hydrophobes,
b) l'amorce la réaction de polymérisation par introduction dans l'émulsion formée en a), d'un initiateur de radicaux libres puis on la laisse se dérouler, et
c) l'on concentre par distillation, le milieu réactionnel issu de l'étape b), jusqu'à élimination complète de ladite huile volatile.

Les huiles volatiles appropriées à la mise en oeuvre du procédé tel que défini ci-dessus, sont par exemple des isoparaffines légères comportant de 8 à 11 atomes de carbone comme par exemple ceux vendues sous les noms ISOPAR™ G, ISOPAR™ L ou ISOPAR™ H ou ISOPAR™ J.

Selon une mise en oeuvre préférée du procédé tel que défini précédemment, la réaction de polymérisation est amorcée par un couple oxydoréducteur, tel que le couple hydroperoxyde de cumène - métabisulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

Lorsque l'étape c) est terminée, on introduit si désiré un ou plusieurs agents émulsifiants de type huile dans eau à une température inférieure à 50°C.

L'invention a aussi pour objet l'utilisation de la composition telle que définie précédemment pour préparer une composition topique cosmétique, dermopharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Une composition topique selon l'invention comporte habituellement entre 0,1 % et 10 % en poids de l'agent épaississant défini ci-dessus. Le pH de la composition topique est de préférence supérieur ou égal à 5.

La composition topique peut en outre comporter des composés classiquement compris dans ce type de compositions, par exemple des parfums, des conservateurs, des colorants, des émollients ou des tensioactifs.

Selon encore un autre aspect, l'invention concerne l'utilisation du nouvel agent épaississant conforme à l'invention, mentionné ci-dessus, pour épaissir et émulsionner une composition topique comprenant au moins une phase aqueuse.

La composition selon l'invention est un substitut intéressant à celles vendues sous les noms SEPIGEL™ 305, SEPIGEL™ 501, SIMULGEL™ EG, SIMULGEL™ NS ou SIMULGEL™ 600 par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings. Elle peut aussi être mise en oeuvre avec lesdits SEPIGEL ou SIMULGEL.

Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2734 496, avec les agents tensioactifs décrits dans WO 93/08204.

Elle est particulièrement compatible avec le MONTANOV™ 68, le MONTANOV™ 82, le MONTANOV™ 202 ou le SEPIPERL™ N. Elle peut également être utilisée dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316.

Elle peut également être utilisée pour former des gels aqueux à pH acide cosmétiquement ou physiologiquement acceptable, tels que ceux décrit dans WO 93/07856 ; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019. ou encore dans les shampooings ou après-shampooings tels que décrits et revendiqués dans WO 92/21316 ou enfin en association avec un homopolymère anionique tels que le CARBO-POL™ pour former des produits de traitement des cheveux comme ceux décrits dans DE 195 23596 ou en association avec d'autres polymères épaississants.

La composition selon l'invention est également compatible avec les principes actifs tels que par exemple, les agents auto-bronzants comme le dihydroxyacétone (DHA) ou les agents anti-acné ; elle peut donc être introduite dans des compositions auto-bronzantes comme celles revendiquées dans EP 0 715 845, EP 0 604249, EP 0576188 ou dans WO 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou dans WO 98/09611.

Lorsque la composition telle que définie précédemment est destinée au traitement de cheveux, elle comprend plus particulièrement un latex inverse de polymère cationique objet de la présente invention.

Lorsque la composition telle que définie précédemment est destinée au traitement de la peau et/ou des muqueuses, elle comprend plus particulièrement un latex inverse de polymère anionique objet de la présente invention.

Les latex inverse objet de la présente invention peuvent être utilisés comme épaississant de pâtes d'impression textile;

Les exemples qui suivent ont pour but d'illustrer la présente invention.

### EXEMPLE 1 : Latex inverse du copolymère AM/AA/(ALE-4OE) réticulé au MBA (Epaississant anionique - composition 1)

a) - Dans un premier bêcher, on introduit successivement sous agitation :
   - 106,5 g d'une solution commerciale d'acrylamide (AM) à 50 % (massique ),
   - 162,0 g d'acide acrylique glacial (AA),
   - 98,1 g d'une solution d'ammoniaque à 29,3 % en poids,
   - 0,047 g de méthylène bis(acrylamide) (MBA),
   - 0,45 g d'une solution commerciale à 40 % du diéthylènetriaminepentaacétate de sodium,
   - de l'eau permutée jusqu'à 680 g.
b) - Dans un deuxième bêcher, on prépare une phase organique en mélangeant :
   - 121 g de polyisobutène,
   - 28 g de MARCOL™ 52,
   - 99 g d'ISOPAR™ H,
   - 17 g de MONTANE™ 70,
   - 3 g d'HYPERMER™ 2296,
   - 5 g de SIMALINE™IE 200,
   - 1,2 g d'acrylate de lauryle tétraéthoxylé (commercial) (ALE-4OE),
   - 0,1 g d'AIBN.
c) - La phase aqueuse est alors introduite dans la phase organique sous agitation et puis la pré-émulsion ainsi obtenue est soumise à une agitation mécanique violente à l'aide d'une turbine de type Silverson de manière à créer une émulsion fine sous barbotage d'azote.
d) - Après refroidissement à environ 8 °C, la réaction de polymérisation est initiée à l'aide du couple oxydoréducteur : hydroperoxyde de cumène / métabisulfite de sodium.
e) - Une fois la réaction de polymérisation terminée, on retire par distillation sous vide l'ISOPAR™ H et la quasi-totalité de l'eau.
f) - On obtient après introduction de 2 % de MONTANOX™ 20 et de 4 % de Laureth 7, un latex inverse épaississant anionique contenant environ 63 % de polymère. Le produit obtenu est peu visqueux, son pouvoir épaississant est important et il s'inverse facilement. Sa teneur en eau mesurée par une titrimétrie Karl-Fisher est de 1,8 % en poids.

### Mesures de viscosité (viscosimètre Brookfield RVT)

A - On mesure les viscosités d'une solution aqueuse comprenant 2 % en poids du latex inverse concentré obtenu et de solutions aqueuses contenant 2 % en poids dudit latex inverse et 0,1%, 1 % et 5 % en poids du chlorure de sodium.

| | Mobile (M) ; Vitesse de rotation du mobile (V) (en tour par minute) | Viscosité en mPas |
|---|---|---|
| Latex inverse | | |
| Solution aqueuse à 2 % en poids | M 6, 5 rpm | 79 400 |
| Solution aqueuse à 2 % en poids + 0,1 % en poids de NaCl | M 6, 5 rpm | 45 200 |
| Solution aqueuse à 2 % en poids + 1 % en poids de NaCl | M 3, 5 rpm | 3 300 |
| Solution aqueuse à 2 % en poids + 5 % en poids de NaCl | M 3, 5 rpm | Nd |

| | | |
|---|---|---|
| nd : non déterminée | | |

### EXEMPLE 2 : Latex inverse du copolymère AM/AA/(ALE-4OE) réticulé au MBA (Epaississant anionique - composition 2)

a) - Dans un premier bêcher, on introduit successivement sous agitation :
   - 106,5 g d'une solution commerciale d'acrylamide (AM) à 50 % (massique),
   - 162,0 g d'acide acrylique glacial (AA),
   - 98,1 g d'une solution d'ammoniaque à 29,3 % en poids,
   - 0,047 g de méthylène bis(acrylamide) (MBA),
   - 0,45 g d'une solution commerciale à 40 % du diéthylènetriaminepentaacétate de sodium,
   - de l'eau permutée jusqu'à 680 g.
b) - Dans un deuxième bêcher, on prépare une phase organique en mélangeant :
   - 121 g de Polyisobutène,
   - 28 g de MARCOL™ 52,
   - 99 g d'ISOPAR™ H,
   - 17 g de MONTANE™ 70,
   - 3 g d'HYPERMER™ 2296,
   - 5 g de SIMALINE™IE 200,
   - 3,0g d'acrylate de lauryle tétraéthoxylé (commercial) (ALE-4OE),
   - 0,1 g d'AIBN.
c) - La phase aqueuse est alors introduite dans la phase organique sous agitation et puis la pré-émulsion ainsi obtenue est soumise à une agitation mécanique violente à l'aide d'une turbine de type Silverson de manière à créer une émulsion fine sous barbotage d'azote.
d) - Après refroidissement à environ 8 °C, la réaction de polymérisation est initiée à l'aide du couple oxydoréducteur : hydroperoxyde de cumène / métabisulfite de sodium.
e) - Une fois la réaction de polymérisation terminée, on retire par distillation sous vide l'ISOPAR™ H et la quasi-totalité de l'eau.
f) - On obtient après introduction de 2 % de MONTANOX™ 20 et 4 % de laureth 7 un latex inverse épaississant anionique contenant environ 63 % de polymère. Le produit obtenu est peu visqueux, son pouvoir épaississant est important et il s'inverse facilement. Sa teneur en eau mesurée par une titrimétrie Karl-Fisher est de 2 % en poids.

### Mesures de viscosité (viscosimètre Brookfield RVT)

A - On mesure les viscosités d'une solution aqueuse comprenant 2 % en poids du latex inverse concentré obtenu et de solutions aqueuses contenant 2 % en poids dudit latex inverse et 0,1 %, 1 % et 5 % en poids du chlorure de sodium.

| | Mobile (M) ; Vitesse de rotation du mobile (V) (en tour par minute) | Viscosité en mPas |
|---|---|---|
| Latex inverse | | |
| Solution aqueuse à 2 % en poids | M 6, 5 rpm | 74 600 |
| Solution aqueuse à 2 % en poids + 0,1 % en poids de NaCl | M 6, 5 rpm | 43 600 |
| Solution aqueuse à 2 % en poids + 1 % en poids de NaCl | M 3, 5 rpm | 6 060 |
| Solution aqueuse à 2 % en poids + 5 % en poids de NaCl | M 3, 5 rpm | Nd |

| | | |
|---|---|---|
| nd : non déterminée | | |

### EXEMPLE 3 : Latex inverse du copolymère AM/AA/(ALE-4OE) (Epaississant anionique - composition 3)

On procède comme à l'exemple précédent mais dans ce cas on utilise 6 g d'acrylate de lauryle tétraéthoxylé et on n'introduit pas de méthyléne bis acrylamide.

On obtient après introduction de 2 % de MONTANOX™ 20 et de 4 % de Laureth 7 un latex inverse épaississant anionique contenant environ 63 % de polymère. Le produit obtenu est peu visqueux , son pouvoir épaississant est important et il s'inverse facilement. Sa teneur en eau mesurée par une titrimétrie Karl-Fisher est de 2,3 % en poids.

### Mesures de viscosité (viscosimètre Brookfield RVT)

A - On mesure les viscosités d'une solution aqueuse comprenant 2 % en poids du latex inverse concentré obtenu et de solutions aqueuses contenant 2 % en poids dudit latex inverse et 0,1 %, 1 % et 5 % en poids du chlorure de sodium.

| | Mobile (M) ; Vitesse de rotation du mobile (V) (en tour par minute) | Viscosité en mPas |
|---|---|---|
| Latex inverse | | |
| Solution aqueuse à 2% en poids | M 6 ; 5 rpm | 37 000 |
| Solution aqueuse à 2% en poids + 0,1 % en poids de NaCl | M 6; 5 rpm | 26 800 |
| Solution aqueuse à 2% en poids + 1 % en poids de NaCl | M 3 ; 5 rpm | 12 100 |
| Solution aqueuse à 2% en poids + 5 % en poids de NaCl | M 3 ; 5 rpm | 7 300 |

### EXEMPLE 4 : Latex inverse du copolymère AM/AA/(ALE-4OE) réticulé au MBA (Epaississant anionique - composition 4)

On procède comme précédemment mais on utilise 6 g d'acrylate de lauryle tétraéthoxylé (commercial) (ALE-4OE et 0,012 g de méthylène bis(acrylamide) (MBA).
f) - On obtient après introduction de 2 % de MONTANOX™ 20 et 4 %de Laureth 7 un latex inverse épaississant anionique contenant environ 63 % de polymère. Le produit obtenu est peu visqueux, son pouvoir épaississant est important et il s'inverse facilement. Sa teneur en eau mesurée par une titrimétrie Karl-Fisher est de 2,2 % en poids.

### Mesures de viscosité (viscosimètre Brookfield RVT)

A - On mesure les viscosités d'une solution aqueuse comprenant 2 % en poids du latex inverse concentré obtenu et de solutions aqueuses contenant 2 % en poids dudit latex inverse et 0,1 %, 1 % et 5 % en poids du chlorure de sodium.

| | Mobile (M) ; Vitesse de rotation du mobile (V) (en tour par minute) | Viscosité en mPas |
|---|---|---|
| Latex inverse | | |
| Solution aqueuse à 2 % en poids | M 6 ; 5 rpm | 75 000 |
| Solution aqueuse à 2 % en poids + 0,1 % en poids de NaCl | M 6 ; 5 rpm | 52 400 |
| Solution aqueuse à 2 % en poids + 1 % en poids de NaCl | M 3 ; 5 rpm | 13 700 |
| Solution aqueuse à 2 % en poids + 5 % en poids de NaCl | M 3 ; 5 rpm | 3000 |

### EXEMPLE 5 : Latex inverse du copolymère AM/AA/(ALE-4OE) réticulé au MBA (Epaississant anionique - composition 5)

On procède comme précédemment mais on introduit 1,2 g d'acrylate de lauryle tétraéthoxylé (commercial) (ALE-4OE) et 0,14 g de méthylène bis(acrylamide) (MBA).

On obtient après introduction de 2 % de MONTANOX™ 20 et de 4 % de Laureth 7 un latex inverse épaississant anionique contenant environ 63 % de polymère. Le produit obtenu est peu visqueux, son pouvoir épaississant est important et il s'inverse facilement. Sa teneur en eau mesurée par une titrimétrie Karl-Fisher est de 2,8 % en poids.

### Mesures de viscosité (viscosimètre Brookfield RVT)

A - On mesure les viscosités d'une solution aqueuse comprenant 2% en poids du latex inverse concentré obtenu et de solutions aqueuses contenant 2 % en poids dudit latex inverse et 0,1 %, 1 % et 5 % en poids du chlorure de sodium.

| | Mobile (M) ; Vitesse de rotation du mobile (V) (en tour par minute) | Viscosité en mPas |
|---|---|---|
| Latex inverse | | |
| Solution aqueuse à 2 % en poids | M 6 ; 5 rpm | 160 000 |
| Solution aqueuse à 2 % en poids + 0,1 % en poids de NaCl | M 6 ; 5 rpm | 80 600 |
| Solution aqueuse à 2 % en poids + 1 % en poids de NaCl | M 3 ;5 rpm | 1 000 |
| Solution aqueuse à 2 % en poids + 5 % en poids de NaCl | M 3 ; 5 rpm | nd |

| | | |
|---|---|---|
| nd : non déterminée | | |

### EXEMPLE 6 : Latex inverse du terpolymère AM/ATBS/AA/(ALE-4OE) (Epaississant anionique - composition 6)

a) - Dans un premier réacteur, on introduit successivement sous agitation :
   - 227,5 kg d'une solution commerciale d'acrylamide (AM) à 50 % (massique),
   - 308,1 kg d'une solution commerciale à 55 % du sel de sodium de l'acide 2-acrylamido 2-méthyl propanesulfonique (ATBS),
   - 8,8 kg d'acide acrylique (AA),
   - 0,032 kg de méthylène bis(acrylamide) (MBA),
   - 0,37 kg d'une solution commerciale à 40 % du diéthylènetriaminepentaacétate de sodium,
   - le pH est ajusté à 6,2 à l'aide d'hydroxyde de sodium ;
   - de l'eau permutée de manière à amener la masse totale à 564,3 kg.
b) - Dans un deuxième réacteur on prépare une phase organique en mélangeant :
   - 107,6 kg de Polyisobutène
   - 25 kg de MARCOL™ 52
   - 74,5 kg d'ISOPAR™ H
   - 14,1 kg de MONTANE™ 70
   - 2,5 kg d'HYPERMER™ 2296
   - 4,1 kg de SIMALINE™IE 200
   - 4,8 kg d'acrylate de lauryle tétraéthoxylé
c) - La phase aqueuse est alors introduite dans la phase organique sous agitation et puis la pré-émulsion ainsi obtenue est soumise à une agitation mécanique violente à l'aide d'une turbine de type Silverson de manière à créer une émulsion fine sous barbotage d'azote.
d) - Après refroidissement à environ 8°C, la réaction de polymérisation est initiée à l'aide du couple oxydoréducteur : persulfate d'ammonium / métabisulfite de sodium.
e) - Une fois la réaction de polymérisation terminée, on retire par distillation sous vide l'ISOPAR™ G et la quasi-totalité d'eau.
f) - On obtient après introduction de 5 % de MONTANOX™ 20 un latex inverse épaississant anionique contenant environ 63 % de polymère. Le produit obtenu est peu visqueux, son pouvoir épaississant est important et il s'inverse facilement. Sa teneur en eau mesurée par une titrimétrie Karl-Fisher est de 3,1 % en poids.

### Mesures de viscosité (viscosimètre Brookfield RVT)

| | Mobile (M) ; Vitesse de rotation du mobile (V) (en tour par minute) | Viscosité en mPas |
|---|---|---|
| Latex inverse | M 4 ; V : 20 | 1450 |
| Solution aqueuse à 2% en poids | M 6 ; V : 5 | 120 000 |
| Solution aqueuse à 2% en poids + 0,1 % en poids de NaCl | M 6 ; V : 5 | 34 000 |

### EXEMPLE 7 : Latex inverse du terpolymère AA/ATBS/ALE 4OE réticulé au MBA (composition 7)

a) - Dans un premier bêcher, on introduit successivement sous agitation :
   - 560 g d'une solution commerciale à 55 % (massique) du sel de sodium de l'acide 2 -acrylamido.2-méthyl propanesulfonique (ATBSNa)
   - 73,3 g d'une solution glacial d'acide acrylique.
   - 40,6 g d'une solution aqueuse à 50 % d'hydroxyde de sodium
   - 0,073 g de méthylène bis(acrylamide) (MBA).
   - 0,45 g d'une solution commerciale à 40 % du diéthylènetriaminepentaacétate de sodium
   - le pH est ajusté à 5,0 à l'aide d'acide 2-acrylamido 2-méthyl propanesulfonique en poudre
   - de l'eau permutée de manière à amener la masse totale à 682 g
b) - Dans un deuxième réacteur on prépare une phase organique en mélangeant :
   - 130 g de Polyisobutène
   - 30 g de MARCOL™ 52
   - 90 g d'ISOPAR™ H
   - 17 g de MONTANE™ 70
   - 3,0 g d'HYPERMER™ 2296
   - 5,0 g de SIMALINE™ IE 200
   - 9,5 g d'acrylate de lauryle tétraéthoxylé
c) - La phase aqueuse est alors introduite dans la phase organique sous agitation et puis la pré-émulsion ainsi obtenue est soumise à une agitation mécanique violente à l'aide d'une turbine de type Silverson de manière à créer une émulsion fine sous barbotage d'azote.
d) - Après refroidissement à environ 8°C, la réaction de polymérisation est initiée à l'aide du couple oxydoréducteur : persulfate d'ammonium / métabisulfite de sodium.
e) - Une fois la réaction de polymérisation terminée, on retire par distillation sous vide l'ISOPAR™ H et la quasi-totalité d'eau.
f) - On obtient après introduction de 2 % de MONTANOX™ 20 et 4% de Laureth 7 un latex inverse épaississant anionique contenant environ 63 % de polymère. Le produit obtenu est peu visqueux, son pouvoir épaississant est important et il s'inverse facilement. Sa teneur en eau mesurée par une titrimétrie Karl-Fisher est de 4 % en poids.

### Mesures de viscosité (viscosimètre Brookfield RVT)

| | Mobile (M) ; Vitesse de rotation du mobile (V) (en tour par minute) | Viscosité en mPas |
|---|---|---|
| Latex inverse | M 4; V : 20 | 4 000 |
| Solution aqueuse à 2% en poids | M 6; V : 5 | 94 000 |
| Solution aqueuse à 2% en poids + 0,1 % en poids de NaCl | M 6; V : 5 | 38 600 |

### Exemple 8 : Latex inverse du terpolymère AM/AA/ (MAS- 20 OE) réticulé au MBA (composition 8)

On procède comme à l'exemple 1 mais on utilise 3,4 g de méthacrylate de stéaryle éthoxylé à 20 moles (MAS-20 OE) et 0,034 g de méthylène -bis-acrylamide. On obtient ainsi un latex inverse épaississant dont les performances sont récapitulées ci-dessous.

| | Mobile (M) ; Vitesse de rotation du mobile (V) (en tour par minute) | Viscosité en mPas |
|---|---|---|
| Latex inverse | | |
| Solution aqueuse à 2 % en poids | M 6 ;5 rpm | 87 000 |
| Solution aqueuse à 2 % en poids + 0,1 % en poids de NaCl | M 6 ; 5 rpm | 49 800 |
| Solution aqueuse à 2 % en poids + 1 % en poids de NaCl | M 3 ;5 rpm | 2600 |
| Solution aqueuse à 2 % en poids + 5 % en poids de NaCl | M 3 ;5 rpm | 120 |

### Exemple 9 : Latex inverse du copolymère AM/AA (MBE - 25 OE) réticulé au MBA (composition 9)

On procède comme à l'exemple 8 mais on utilise 3,3 g de méthacrylate de béhènyle éthoxylé à 25 moles d'oxyde d'éthylène (MBE - 25 OE) à la place du MAS - 20 OE.

| | Mobile (M) ; Vitesse de rotation du mobile (V) (en tour par minute) | Viscosité en mPas |
|---|---|---|
| Latex inverse | | |
| Solution aqueuse à 2 % en poids | M 6 ;5 rpm | 70 000 |
| Solution aqueuse à 2 % en poids + 0,1 % en poids de NaCl | M 6 ; 5 rpm | 42 400 |
| Solution aqueuse à 2% en poids + 1 % en poids de NaCl | M 3 ;5 rpm | 5 100 |
| Solution aqueuse à 2% en poids + 5 % en poids de NaCl | M 3 ;5 rpm | 180 |

### Exemples de formulations cosmétiques

### Exemple 10 Crème de soin

| | | |
|---|---|---|
| DOW CORNING™ 345 : | | 10% |
| Composition 2: | | 0,8 % |
| MONTANOV™ 68: | | 4,5 % |
| Conservateur : | | 0,65 % |
| Lysine : | | 0,025 % |
| EDTA (sel disodique) : | | 0,05 % |
| KETROL™ T : | | 0,2 % |
| Glycérine: | | 3% |
| Eau: | qsp | 100 % |

### Exemple 11 : Crème de soin

| | | |
|---|---|---|
| DOW CORNING™ 345 : | | 10 % |
| Composition 4 : | | 0,8 % |
| MONTANOV™ 68: | | 4,5 % |
| Perfluoropolyméthylisopropyléther : | | 0,5 % |
| Conservateur : | | 0,65 % |
| Lysine : | | 0,025 % |
| EDTA (sel disodique) : | | 0,05 % |
| PEMULEN™ TR: | | 0,2 % |
| Glycérine : | | 3 % |
| Eau: | qsp | 100% |

### Exemple 12 : Baume après-rasage

### FORMULE

| | | | |
|---|---|---|---|
| A | Composition 3 : | | 1,5 % |
| | Eau: | qsp | 100 % |
| B | MICROPEARL™ M100 : | | 5,0 % |
| | SEPICIDE™ CI : | | 0,50 % |
| | Parfum : | | 0,20 % |
| | Ethanol à 95° : | | 10,0 % |

### MODE OPERATOIRE

### Ajouter B dans A.

### Exemple 13 : Emulsion satinée pour le corps

### FORMULE

| | | |
|---|---|---|
| A | SIMULSOL™ 165 : | 5,0 % |
| | LANOL™ 1688 : | 8,50 % |
| | Beurre de Karité : | 2 % |
| | Huile de paraffine: | 6,5 % |
| | LANOL™ 14 M : | 3 % |
| | LANOL™ S : | 0,6 % |
| B | Eau: | 66,2% |
| C | MICROPEARL™ M100 : | 5 % |
| D | Composition 5 : | 3% |
| E | SEPICIDE™ CI : | 0,3 % |
| | SEPICIDE™ HB : | 0,5 % |
| | MONTEINE™ CA : | 1 % |
| | Parfum : | 0,20 % |
| | Acétate de vitamine E : | 0,20 % |
| | pyrolidinonecarboxylate de sodium : | 1% (agent hydratant) |

### MODE OPERATOIRE

Ajouter C dans B, émulsionner B dans A à 70°C; puis ajouter D à 60°C puis E à 30°C.

### Exemple 14 : Lait corporel

### FORMULE

| | | | |
|---|---|---|---|
| A | SIMULSOL™ 165 : | | 5,0 % |
| | LANOL™ 1688 : | | 12,0 % |
| | LANOL™ 14 M : | | 2,0% |
| | Alcool cétylique : | | 0,3 % |
| | SCHERCEMOL™ OP : | | 3 % |
| B | Eau : | q.s.p. | 100% |
| C | Composition 4: | | 0,35 % |
| D | SEPICIDE™ CI : | | 0,2 % |
| | SEPICIDE™ HB : | | 0,5 % |
| | Parfum : | | 0,20 % |

### MODE OPERATOIRE

Emulsionner B dans A vers 75°C ; ajouter C vers 60°C, puis D vers 30°C

### Exemple 15 : crème H/E

### FORMULE

| | | | |
|---|---|---|---|
| A | SIMULSOL™ 165 : | | 5,0 % |
| | LANOL™ 1688: | | 20,0 % |
| | LANOL™ P: | | 1,0 % |
| B | Eau: | q.s.p. | 100 % |
| C | Composition 2: | | 2,50 % |
| D | SEPICIDE™ CI : | | 0,20 % |
| | SEPICIDE™ HB : | | 0,30 % |

### MODE OPERATOIRE

Introduire B dans A vers 75°C ; ajouter C vers 60°C, puis D vers 45°C

### Exemple 16 : gel solaire non gras

### FORMULE

| | | | |
|---|---|---|---|
| A | Composition 5 : | | 3,00 % |
| | Eau : | | 30 % |
| B | SEPICIDE™ C : | | 0,20 % |
| | SEPICIDE™ HB : | | 0,30 % |
| | Parfum : | | 0,10 % |
| C | Colorant : | | qs |
| | Eau: | | 30 % |
| | D MICROPEARL™ M100 : | | 3,00 % |
| | Eau : | q.s.p. | 100 % |
| E | Huile de silicone : | | 2,0 % |
| | PARSOL™ MCX : | | 5,00 % |

### MODE OPERATOIRE

Introduire B dans A; ajouter C puis D, puis E.

### Exemple 17 : Lait solaire

### FORMULE

| | | | |
|---|---|---|---|
| A | SEPIPERL™ N : | | 3,0 % |
| | Huile de sésame : | | 5,0 % |
| | PARSOL™ MCX : | | 5,0 % |
| | Carraghénane λ : | | 0,10 % |
| B | Eau : | q.s.p. | 100 % |
| C | Composition 3 : | | 0,80 % |
| D | Parfum : | | q.s. |
| | Conservateur : | | q.s. |

### MODE OPERATOIRE

Emulsionner B dans A à 75°C puis ajouter C vers 60°C, puis D vers 30°C et ajuster le pH si nécessaire

### Exemple 18 : Gel de massage

### FORMULE

| | | |
|---|---|---|
| A | Composition 2: | 3,5 % |
| | Eau : | 20,0 % |
| B | Colorant: | 2 gouttes /100 g |
| | Eau : | q.s. |
| C | Alcool: | 10% |
| | Menthol: | 0,10% |
| D | Huile de silicone : | 5,0 % |

### MODE OPERATOIRE

Ajouter B dans A; puis ajouter au mélange, C puis D

### Exemple 19 : gel soin de massage

### FORMULE

| | | | |
|---|---|---|---|
| A | Composition 3 : | | 3,00 % |
| | Eau: | | 30 % |
| B | SEPICIDE™ CI : | | 0,20 % |
| | SEPICIDE™ HB : | | 0,30 % |
| | Parfum : | | 0,05 % |
| C | colorant: | | q.s. |
| | Eau: | q.s.p. | 100 % |
| D | MICROPEARL™ SQL : | | 5,0 % |
| | LANOL™ 1688 : | | 2 % |

### MODE OPERATOIRE

Préparer A ; additionner B, puis C, puis D.

### Exemple 20 : Gel coup d'éclat

### FORMULE

| | | | |
|---|---|---|---|
| A | Composition 4 : | | |
| | Eau : | | 30 % |
| B | ELASTINE HPM : | | 5,0 % |
| C | MICROPEARL™ M 100 : | | 3 % |
| | Eau : | | 5 % |
| D | SEPICIDE™ CI : | | 0,2 % |
| | SEPICIDE™ HB : | | 0,3 % |
| | Parfum : | | 0,06 % |
| | pyrolidinonecarboxylatede sodium à 50 % : | | 1 % |
| | Eau : | q.s.p. | 100 % |

### MODE OPERATOIRE

Préparer A; additionner B, puis C, puis D.

### Exemple 21 : Lait corporel

### FORMULE

| | | | |
|---|---|---|---|
| A | SEPIPERL™ N : | | 3,0 % |
| | Triheptonate de glycérol : | | 10,0 % |
| B | Eau : | q.s.p. | 100 % |
| C | Composition 5 : | | 1,0 % |
| D | Parfum : | | q.s. |
| | Conservateur : | | q.s. |

### MODE OPERATOIRE

Fondre A à environ 75°C . Emulsionner B dans A à 75°C puis ajouter C vers 60°C, puis D.

### Exemple 22 : Emulsion démaquillante à l'huile d'amandes douces

### FORMULE

| | | |
|---|---|---|
| MONTANOV™ 68 : | | 5 % |
| Huile d'amandes douces : | | 5 % |
| Eau: | q.s.p. | 100 % |
| Composition 4 : | | 0,3 % |
| Glycérine: | | 5 % |
| Conservateur : | | 0,2 % |
| Parfum : | | 0,3 % |

### Exemple 23 : Crème hydratante pour peaux grasses

### FORMULE

| | | |
|---|---|---|
| MONTANOV™ 68 : | | 5 % |
| Octanoate de cétylstéaryle : | | 8 % |
| palmitate d'octyle : | | 2 % |
| Eau : | q.s.p. | 100 % |
| Composition 3 : | | 0,6 % |
| MICROPEARL™ M100 : | | 3,0 % |
| Mucopolysaccharides : | | 5 % |
| SEPICIDE™ HB : | | 0,8 % |
| Parfum : | | 0,3 % |

### Exemple 24 : Baume après-rasage apaisant sans alcool

### FORMULE

| | | |
|---|---|---|
| Mélange de lauryl aminoacides : | | 0,1 % à 5 % |
| Aspartate de magnésium et de potassium : | | 0,002 % à 0,5 % |
| LANOL™ 99 : | | 2 % |
| Huile d'amandes douces : | | 0,5 % |
| Eau : | q.s.p. | 100 % |
| Composition 2 : | | 3 % |
| SEPICIDE™ HB : | | 0,3 % |
| SEPICIDE™ CI : | | 0,2 % |
| Parfum : | | 0,4 % |

### Exemple 25 : Crème aux AHA pour peaux sensibles

### FORMULE

| | | |
|---|---|---|
| Mélange de lauryl aminoacides : | 0,1 % à 5 % | |
| Aspartate de magnésium et de potassium : | 0,002 % à 0,5 % | |
| LANOL™ 99 : | | 2 % |
| MONTANOV™ 68 : | | 5,0 % |
| Eau : | q.s.p. | 100 % |
| Composition 2 : | | 1,50 % |
| Acide gluconique : | | 1,50 % |
| Triéthanolamine : | | 0,9 % |
| SEPICIDE™ HB : | | 0,3 % |
| SEPICIDE™ CI : | | 0,2 % |
| Parfum : | | 0,4 % |

### Exemple 26 : Soin apaisant après-soleil

### FORMULE

| | | |
|---|---|---|
| Mélange de lauryl aminoacides : | | 0,1 % à 5 % |
| Aspartate de magnésium et de potassium : | | 0,002 % à 0,5 % |
| LANOL™ 99 : | | 10,0% |
| Eau : | q.s.p. | 100 % |
| Composition 4 : | | 2,50 % |
| SEPICIDE™ HB : | | 0,3 % |
| SEPICIDE™ CI : | | 0,2 % |
| Parfum : | | 0,4 % |
| Colorant : | | 0,03 % |

### Exemple 27 : Lait démaquillant

### FORMULE

| | | |
|---|---|---|
| SEPIPERL™ N : | | 3 % |
| PRIMOL™ 352 : | | 8,0 % |
| Huile d'amandes douces : | | 2 % |
| Eau : | q.s.p. | 100 % |
| Composition 3 : | | 0,8 % |
| Conservateur : | | 0,2 % |

### Exemple 28 : Lait corporel

### FORMULE

| | | |
|---|---|---|
| SEPIPERL™ N : | | 3,5 % |
| LANOL™ 37T : | | 8,0 % |
| SOLAGUM™ L : | | 0,05 % |
| Eau: | q.s.p. | 100 % |
| Benzophénone : | | 2,0 % |
| Diméthicone 350 cPs : | | 0,05 % |
| Composition 5 : | | 0,8 % |
| Conservateur : | | 0,2 % |
| Parfum : | | 0,4% |

### Exemple 29 : émulsion fluide à pH alcalin

| | | |
|---|---|---|
| MARCOL™ 82 : | | 5,0 % |
| NaOH : | | 10,0% |
| Eau: | q.s.p. | 100 % |
| Composition 2 : | | 1,5 % |

### Exemple 30 : Fond de teint fluide

### FORMULE

| | | |
|---|---|---|
| SIMULSOL™ 165 : | | 5,0 % |
| LANOL™ 84D : | | 8,0 % |
| LANOL™ 99 : | | 5,0% |
| Eau: | q.s.p. | 100 % |
| Pigments et charges minérales : | | 10,0% |
| Composition 3 : | | 1,2% |
| Conservateur : | | 0,2 % |
| Parfum : | | 0,4 % |

### Exemple 31 : Lait solaire

### FORMULE

| | | |
|---|---|---|
| SEPIPERL™N : | | 3,5 % |
| LANOL™ 37T : | | 10,0 % |
| PARSOL™ NOX : | | 5,0 % |
| EUSOLEX™ 4360 : | | 2,0 % |
| Eau: | q.s.p. | 100 % |
| Composition 4 : | | 1,8 % |
| Conservateur : | | 0,2 % |
| Parfum : | | 0,4 % |

### Exemple 32 : Gel contour des yeux

### FORMULE

| | | |
|---|---|---|
| Composition 3 : | | 2,0 % |
| Parfum : | | 0,06 % |
| pyrrolidinonecarboxylate de sodium : | | 0,2 % |
| DOW CORNING™ 245 FLuid : | | 2,0 % |
| Eau : | q.s.p. | 100% |

### Exemple 33 : Composition de soin non rincée

### FORMULE

| | | |
|---|---|---|
| Composition 4: | | 1,5 % |
| Parfum : | | q.s. |
| Conservateur : | | q.s. |
| DOW CORNING™ X2 8360 : | | 5,0 % |
| DOW CORNING™ Q2 1401: | | 15,0 % |
| Eau: | q.s.p. | 100% |

### Exemple 34 : Gel amincissant

| | | |
|---|---|---|
| Composition 5 : | | 5 % |
| Ethanol : | | 30 % |
| Menthol : | | 0,1 % |
| Caféine : | | 2,5 % |
| Extrait de ruscus : | | 2 % |
| Extrait de lierre : | | 2 % |
| SEPICIDE™ HB | | 1 % |
| Eau | q.s.p. | 100 % |

### Exemple 35 : Baume après-rasage apaisant sans alcool

### FORMULE

| | | | |
|---|---|---|---|
| A | LIPACIDE™ PVB : | | 1,0 % |
| | LANOL™ 99 : | | 2,0 % |
| | Huile d'amandes douces : | | 0,5 % |
| B | Composition 3 : | | 3,5 % |
| C | Eau: | q.s.p. | 100 % |
| D | Parfum : | | 0,4 % |
| | SEPICIDE™ HB : | | 0,4 % |
| | SEPICIDE™ CI: | | 0,2 % |

### Exemple 36: Gel rafraîchissant après-rasage

### FORMULE

| | | | |
|---|---|---|---|
| A | LIPACIDE™ PVB : | | 0,5 % |
| | LANOL™ 99 : | | 5,0 % |
| | Composition 2 : | | 2,5 % |
| B | eau: | q.s.p. | 100 % |
| C | MICROPEARL™ LM : | | 0,5 % |
| | Parfum : | | 0,2 % |
| | SEPICIDE™ HB : | | 0,3 % |
| | SEPICIDE™ CI : | | 0,2 % |

### Exemple 37 : Soin pour les peaux grasses

### FORMULE

| | | | |
|---|---|---|---|
| A | MICROPEARL™ M310 : | | 1,0 % |
| | Composition 4 : | | 5,0 % |
| | Isononanoate d'octyle : | | 4,0 % |
| B | Eau: | q.s.p. | 100 % |
| C | SEPICONTROL™ A5 : | | 4,0 % |
| | Parfum : | | 0,1 % |
| | SEPICIDE™ HB : | | 0,3 % |
| | SEPICIDE™ CI : | | 0,2 % |
| D | CAPIGEL™ 98 : | | 0,5 % |
| | Eau: | | 10% |

### Exemple 38 : Crème aux AHA

### FORMULE

| | | | |
|---|---|---|---|
| A | MONTANOV™ 68: | | 5,0 % |
| | LIPACIDE™ PVB : | | 1,05 % |
| | LANOL™ 99 : | | 10,0 % |
| B | Eau: | q.s.p. | 100 % |
| | Acide gluconique : | | 1,5 % |
| | TEA (triéthanolamine) : | | 0,9 % |
| C | Composition 5 : | | 1,5 % |
| D | Parfum: | | 0,4 % |
| | SEPICIDE™ HB : | | 0,2 % |
| | SEPICIDE™ CI : | | 0,4 % |

### Exemple 39 : Autobronzant non gras pour visage et corps

### FORMULE

| | | | |
|---|---|---|---|
| A | LANOL™ 2681 : | | 3,0 % |
| | Composition 4 | | 2,5 % |
| B | Eau: | q.s.p. | 100% |
| | Dihydroxyacétone: | | 3,0% |
| C | Parfum : | | 0,2 % |
| | SEPICIDE™ HB : | | 0,8 % |
| | hydroxyde de sodium : | qs | pH = 5 % |

### Exemple 40 : Lait solaire au monoï de Tahiti

### FORMULE

| | | | |
|---|---|---|---|
| A | Monoï de Tahiti: | | 10 % |
| | LIPACIDE™ PVB : | | 0,5 % |
| | Composition 2 : | | 2,2 % |
| B | Eau : | q.s.p. | 100 % |
| C | Parfum : | | 0,1 % |
| | SEPICIDE™ HB : | | 0,3 % |
| | SEPICIDE™ CI : | | 0,1 % |
| | PARSOL™ MCX: | | 4,0 % |

### Exemple 41 : Soin solaire pour le visage

### FORMULE

| | | | |
|---|---|---|---|
| A | DC™ 1501 : | | 4,0% |
| | Composition 3 : | | 3,5 % |
| B | Eau : | q.s.p. | 100% |
| C | Parfum : | | 0,1% |
| | SEPICIDE™ HB : | | 0,3 % |
| | SEPICIDE™ CI : | | 0,21 % |
| | Méthoxycinnamate d'octyle : | | 5,0 % |
| | Micatitane : | | 2,0 % |
| | Acide lactique : | q.s.p. | pH = 6,5 |

### Exemple 42 Emulsion bronzante sans soleil

### FORMULE

| | | | |
|---|---|---|---|
| A | LANOL™ 99 : | | 15 % |
| | MONTANOV™ 68 : | | 5,0 % |
| | Paraméthoxycinnamate d'octyle : | | 3,0 % |
| B | Eau : | q.s.p. | 100 % |
| | Dihydroxyacétone : | | 5,0 % |
| | Phosphate monosodique : | | 0,2 % |
| C | Composition 4 : | | 0,5 % |
| D | Parfum : | | 0,3 % |
| | SEPICIDE™ HB: | | 0,8 % |
| | hydroxyde de sodium : | q.s. | pH = 5. |

### Exemple 43 : Gel brillance

| | | |
|---|---|---|
| Composition 5 : | | 1,5 % |
| Silicone volatile : | | 25 % |
| Monopropylèneglycol | | 25 % |
| Eau déminéralisée : | | 10 % |
| Glycérine: | qsp | 100% |

### Exemple 44 : Gel amincissant

| | | |
|---|---|---|
| Composition 4 : | | 1,5 % |
| LANOL™ 99 : | | 2 % |
| Caféine : | | 5 % |
| Ethanol: | | 40 % |
| MICROPEARL™ LM : | | 2 % |
| Eau déminéralisée : | qsp | 100 % |
| Conservateur parfum : | | qs |

### Exemple 45 : Lait démaquillant

| | | |
|---|---|---|
| SIMULSOL™ 165 : | | 4 % |
| MONTANOV™ 202 : | | 1 % |
| Caprylate-caprate triglycéride : | | 15 % |
| PECOSIL™ DCT : | | 1 % |
| Eau déminéralisée : | | qs |
| CAPIGEL™ 98 : | | 0,5 % |
| Composition 5 : | | 1% |
| PROTEOL™ OAT : | | 2 % |
| Hydroxyde de sodium : | qsp | pH = 7 |

### Exemple 46 : Masque crème "rince off" restructurant pour cheveux stressés et fragilisés

### Formule

| | | |
|---|---|---|
| KETROL™ T : | | 0,5% |
| PECOSIL™ SPP50 : | | 0,75% |
| N-cocoyl aminoacides : | | 0,70% |
| Butylèneglycol : | | 3,0% |
| Composition 1 : | | 3,0% |
| MONTANOV™ 82 : | | 3,0% |
| Huile de jojoba : | | 1,0% |
| LANOL™ P : | | 6,0% |
| AMONYL™ DM : | | 1,0% |
| LANOL™ 99 : | | 5,0% |
| SEPICIDE™ HB : | | 0,3% |
| SEPICIDE™CI : | | 0,2% |
| Parfum : | | 0,2% |
| Eau : | qsp | 100% |

### Exemple 47 : Crème solaire

| | | |
|---|---|---|
| SIMULSOL™ 165 : | | 3 % |
| MONTANOV™ 202 : | | 2 % |
| Benzoate C12-C15 : | | 8 % |
| PECOSIL™ PS 100 : | | 2 % |
| Diméthicone : | | 2 % |
| DOW CORNING™ 345 : | | 5 % |
| para-méthoxy cinnamate d'octyle : | | 6 % |
| Benzophénone-3 : | | 4 % |
| Oxyde de Titane : | | 8 % |
| KETROL™ T : | | 0,2 % |
| Butylèneglycol : | | 5 % |
| Eau déminéralisée: | qsp | 100 % |
| Composition 2 : | | 1,5 % |
| Conservateur, parfum : | | qs |

### Exemple 48 : Gel de soin peaux mixtes

| | | |
|---|---|---|
| Composition 3 : | | 4 % |
| Squalane végétal: | | 5 % |
| Dimethicone : | | 1,5 % |
| SEPICONTROL™ A5 : | | 4 % |
| KETROL™ T : | | 0,3 % |
| Eau: | qsp | 100 % |
| Conservateur, Parfum : | | |

### Exemple 49 : Lotion capillaire

| | | |
|---|---|---|
| Butylène glycol: | | 3,0% |
| Composition 6 : | | 3% |
| SIMULSOL™ 1293 : | | 3,0% |
| Acide lactique : | qs | pH = 6 |
| SEPICIDE™ HB : | | 0,2% |
| SEPICIDE™CI: | | 0,3% |
| Parfum : | | 0,3% |
| Eau : | qs | 100% |

### Exemple 50 : Shampooing protecteur et relaxant

| | | |
|---|---|---|
| Amonyl™ 675 SB : | | 5,0% |
| Sodium lauryl éther sulfate à 28% : | | 35,0% |
| composition 6 : | | 3,0% |
| SEPICIDE™ HB: | | 0,5% |
| SEPICIDE™CI: | | 0,3% |
| hydroxyde de sodium : | QS | pH = 7,2 |
| Parfum: | | 0,3% |
| Colorant (FDC bleu 1/jaune 5) : | | QS |
| Eau: | QSP | 100% |

### Exemple 51 : Protecteur "leave-on" ; Soin anti-stress pour cheveux

| | |
|---|---|
| KETROL™ T : | 0,5% |
| mélange de cocoyl aminoacides: | 3,0% |
| Butylèneglycol : | 5,0% |
| DC 1501 : | 5,0% |
| Composition 1 : | 4,0% |
| SEPICIDE™ HB: | 0,5% |
| SEPICIDE™CI: | 0,3% |
| Parfum : | 0,3% |
| Eau : | QSP 100 |

### Exemple 52 : Crème vitaminée

| | | |
|---|---|---|
| SIMULSOL™ 165 : | | 5 % |
| MONTANOV™ 202 : | | 1 % |
| Caprylic/capric triglycérides : | | 20 % |
| Palmitate de vitamine A : | | 0,2 % |
| Acétate de vitamine E : | | 1 % |
| MICROPEARL™ M305 : | | 1,5 % |
| Composition 1 : | | 2 % |
| Eau | qsp | 100 % |
| Conservateur, parfum | | qs |

### Exemple 53 : Crème de soin

| | | |
|---|---|---|
| DOW CORNING™ 345 : | | 10% |
| Composition 1 : | | 0,8% |
| MONTANOV™ 68 : | | 2% |
| alcool stéarylique : | | 1% |
| alcool stéarique : | | 0,5% |
| conservateur : | | 0,65% |
| Lysine : | | 0,025% |
| EDTA (sel disodique) : | | 0,05% |
| KETROL™ T: | | 0,2% |
| Glycérine : | | 3% |
| Eau: | q.s.p. | 100% |

### Exemple 54 : Baume après-rasage

### FORMULE

| | | | |
|---|---|---|---|
| A | Composition 3 : | | 1,5% |
| | Eau: | q.s.p | 100% |
| B | MICROPEARL™ M100 : | | 5,0% |
| | SEPICIDE™ CI : | | 0,50% |
| | Parfum : | | 0,20% |
| | Ethanol 95° : | | 10,0% |

### MODE OPERATOIRE

Ajouter B dans A.

### Exemple 55 : Emulsion satinée pour le corps

### FORMULE

| | | |
|---|---|---|
| A | SIMULSOL™ 165 : | 5,0% |
| | LANOL™ 1688 : | 8,50% |
| | beurre de Karité : | 2% |
| | huile de paraffine : | 6,5% |
| | LANOL™ 14M : | 3% |
| | LANOL™ S : | 0,6% |
| B | eau : | 66,2% |
| C | MICROPEARL™ M100 : | 5% |
| D | Composition 5 : | 3% |
| E | SEPICIDE™ CI: | 0,3% |
| | SEPICIDE™ HB: | 0,5% |
| | AQUAXYL™ : | 3% |
| | Parfum : | 0,20% |
| | acétate de vitamine E : | 0,20% |
| | Sodium pyrolidinonecarboxylate : | 1% |

### MODE OPERATOIRE

Ajouter C dans B, émulsionner B dans A à 70°C, puis ajouter D à 60°C puis E à 30°C.

### Exemple 56 : Crème H/E

### FORMULE

| | | | |
|---|---|---|---|
| A | SIMULSOL™ 165 : | | 5,0% |
| | LANOL™ 1688 : | | 20,0% |
| | LANOL™ P : | | 1,0% |
| B | eau : | q.s.p. | 100% |
| C | Composition 2: | | 2,50% |
| D | SEPICIDE™ CI : | | 0,20% |
| | SEPICIDE™ HB : | | 0,30% |

### MODE OPERATOIRE

Introduire B dans A vers 75°C ; ajouter C vers 60°C, puis D vers 45°C.

### Exemple 57 : Gel solaire non gras

### FORMULE

| | | | |
|---|---|---|---|
| A | Composition 7: | | 3,00% |
| | Eau : | | 30% |
| B | SEPICIDE™ CI : | | 0,20% |
| | SEPICIDE™ HB : | | 0,30% |
| | parfum : | | 0,10% |
| C | colorant: | | q.s |
| | eau: | | 30% |
| | D MICROPEARL™ M100 : | | 3,00% |
| | eau : | q.s.p | 100% |
| E | huile de silicone : | | 2,0% |
| | PARSOL™ MCX : | | 5,00% |

### MODE OPERATOIRE

Introduire B dans A; ajouter C, puis D, puis E.

### Exemple 58 : Lait solaire

### FORMULE

| | | | |
|---|---|---|---|
| A | MONTANOV™ S : | | 3,0% |
| | Huile de sésame : | | 5,0% |
| | PARSOL™ MCX : | | 5,0% |
| | Carraghénane λ : | | 0,10% |
| | B eau : | q.s.p. | 100% |
| C | Composition 1 : | | 0,80% |
| D | Parfum : | | q.s. |
| | Conservateur : | | q.s. |

### MODE OPERATOIRE

Emulsionner B dans A à 75°C puis ajouter C vers 60°C, puis D vers 30°C et ajuster le pH si nécessaire

### Exemple 59 : Gel de massage

### FORMULE

| | | |
|---|---|---|
| A | Composition 8 : | 3,5% |
| | Eau: | 20,0% |
| B | colorant : | 2 gouttes/100g |
| | Eau: | q. s. |
| C | alcool: | 10% |
| | Menthol: | 0,10% |
| D | huile de silicone : | 5,0% |

### MODE OPERATOIRE

Ajouter B dans A, puis ajouter au mélange, C puis D

### Exemple 60 : Fond de teint hydratant et matifiant

### FORMULE

| | | |
|---|---|---|
| A | eau : | 20,0% |
| | Butylène glycol: | 4,0% |
| | PEG-400 : | 4,0% |
| | PECOSIL™ PS100 : | 1,0% |
| | NaOH : | q.s. pH = 9 |
| | Dioxyde de titane : | 7,0% |
| | Talc : | 2,0% |
| | Oxyde de fer jaune : | 0,8% |
| | Oxyde de fer rouge : | 0,3% |
| | Oxyde de fer noir : | 0,05% |
| B | LANOL™ 99: | 8% |
| | Caprylic capric triglycéride | 8% |
| | MONTANOV™ 202 : | 5,00% |
| C | eau : | q.s.p. 100% |
| | MICROPEARL™ M305 : | 2,0% |
| | EDTA tétrasodé : | 0,05% |
| D | DOW CORNING™ 345 : | 4,0% |
| | Gomme de Xanthanse : | 0,2% |
| | Composition 5 : | 0,8% |
| E | SEPICIDE™ HB: | 0,5% |
| | SEPICIDE CI : | 03% |
| | Parfum : | 0,2% |

### MODE OPERATOIRE

préparer à 80°C, les mélanges B + D et A + C, puis mélanger et émulsionner l'ensemble.

### Exemple 61 : Gel coup d'éclat

### FORMULE

| | | | |
|---|---|---|---|
| A | Composition 5 : | | 4% |
| | Eau: | | 30% |
| B | ELASTINE HPM : | | 5,0% |
| C | MICROPEARL™ M100 : | | 3% |
| | Eau: | | 5% |
| D | SEPICIDE™ CI : | | 0,2% |
| | SEPICIDE™ HB : | | 0,3% |
| | Parfum : | | 0,06% |
| | Sodium pyrolidinonecarboxylate 50% : | | 1% |
| Eau: | | q.s.p. | 100% |

### MODE OPERATOIRE

Préparer A ; additionner B, puis C, puis D.

### Exemple 62 : Lait corporel

### FORMULE

| | | |
|---|---|---|
| MONTANOV™ S : | | 3,5% |
| LANOL™ 37T : | | 8,0% |
| SOLAGUM™ L : | | 0,05% |
| Eau: | q.s.p. | 100% |
| Benzophénone 3 : | | 2,0% |
| diméthicone 350cPs : | | 0,05% |
| Composition 4 : | | 0,8% |
| conservateur : | | 0,2% |
| parfum : | | 0,4% |

### Exemple 63 : Emulsion démaquillante à l'huile d'amandes douces

### FORMULE

| | | |
|---|---|---|
| MONTANOV™ 68 : | | 5% |
| huile d'amandes douces : | | 5% |
| eau : | q.s.p. | 100% |
| Composition 3 : | | 0,3% |
| glycérine : | | 5% |
| conservateur : | | 0,2% |
| parfum : | | 0,3% |

### Exemple 64 : Crème hydratante pour peaux grasses

### FORMULE

| | | |
|---|---|---|
| MONTANOV™ 68 : | | 5% |
| Cétylstéaryloctanoate : | | 8% |
| octyl palmitate : | | 2% |
| eau : | q.s.p. | 100% |
| Composition 3 : | | 0,6% |
| MICROPEARL™ M100 : | | 3,0% |
| Mucopolysaccharides : | | 5% |
| SEPICIDE™ HB : | | 0,8% |
| Parfum : | | 0,3% |

### Exemple 65 : Baume après-rasage apaisant sans alcool

### FORMULE

| | | | |
|---|---|---|---|
| A | LIPACIDE™ PVB : | | 1,0% |
| | LANOL™ 99: | | 2,0% |
| | Huile d'amandes douces : | | 0,5% |
| B | Composition 1 : | | 3,5% |
| C | eau: | q.s.p. | 100% |
| D | parfum : | | 0,4% |
| | SEPICIDE™ HB : | | 0,4% |
| | SEPICIDE™ CI : | | 0,2% |

### Exemple 66 : Crème aux AHA pour peaux sensibles

| | | |
|---|---|---|
| Mélange de lauryl aminoacides : | | 0,1% à 5% |
| aspartate de magnésium et de potassium : | 0,002% | à 0,5% |
| LANOL™ 99: | | 2% |
| MONTANOV™ 68: | | 5,0% |
| Eau : | q.s.p. | 100% |
| Composition 4 : | | 1,50% |
| acide gluconique : | | 1,50% |
| tri éthylamine : | | 0,9% |
| SEPICIDE™ HB : | | 0,3% |
| SEPICIDE™ CI : | | 0,2% |
| Parfum : | | 0,4% |

### Exemple 67 : Soin apaisant après soleil

| | | |
|---|---|---|
| Mélange de lauryl aminoacides : | | 0,1% à 5% |
| aspartate de magnésium et de potassium : | | 0,002% à 0,5% |
| LANOL™ 99: | | 10,0% |
| Eau: | q.s.p. | 100% |
| Composition 2 : | | 2,50% |
| SEPICIDE™ HB : | | 0,3% |
| SEPICIDE™ CI : | | 0,2% |
| Parfum : | | 0,4% |
| Colorant : | | 0,03% |

### Exemple 68 : Lait démaquillant

| | | |
|---|---|---|
| SEPIPERL™ N : | | 3% |
| PRIMOL™ 352 : | | 8,0% |
| Huile d'amandes douces : | | 2% |
| Eau: | q.s.p. | 100% |
| Composition 5 : | | 0,8% |
| conservateur : | | 0,2% |

### Exemple 69 : Emulsion fluide à pH alcalin

| | | |
|---|---|---|
| MARCOL™ 82 : | | 5,0% |
| NaOH : | | 10,0% |
| Eau : | q.s.p. | 100% |
| Composition 4 : | | 1,5% |

### Exemple 70 : Fond de teint fluide

| | | |
|---|---|---|
| SIMULSOL™ 165 : | | 5,0% |
| LANOL™ 84D : | | 8,0% |
| LANOL™ 99 : | | 5,0% |
| Eau : | q.s.p. | 100% |
| Pigments et charges minérales : | | 10,0% |
| Composition 5 : | | 1,2% |
| Conservateur : | | 0,2% |
| Parfum : | | 0,4% |

### Exemple 71 : Lait solaire

| | | |
|---|---|---|
| SEPIPERL™ N : | | 3,5% |
| LANOL™ 37T : | | 10,0% |
| PARSOL™ MCX : | | 5,0% |
| EUSOLEX™ 4360 : | | 2,0% |
| Eau : | q.s.p. | 100% |
| Composition 1 : | | 1,8% |
| Conservateur : | | 0,2% |
| Parfum : | | 0,4% |

### Exemple 72 : Gel contour des yeux

| | | |
|---|---|---|
| Composition 2 : | | 2,0% |
| Parfum : | | 0,06% |
| Sodium pyrrolidinonecarboxylate : | | 0,2% |
| DOW CORNING™ 245 Fluid : | | 2,0% |
| Eau : | q. s. p. | 100% |

### Exemple 73 : Composition de soin non rincée

| | | |
|---|---|---|
| Composition 3 : | | 1,5% |
| Parfum : | | q.s |
| Conservateur : | | q. s. |
| DOW CORNING™ X2 8360 : | | 5,0% |
| DOW CORNING™ Q2 1401 : | | 15,0% |
| Eau : | q.s.p. | 100% |

| **Exemple 74 : Gel amincissant** | | |
|---|---|---|
| Composition 6 : | | 5% |
| Ethanol : | | 30% |
| Menthol : | | 0,1% |
| Caféine : | | 2,5% |
| Extraits de ruscus : | | 2% |
| Extrait de lierre : | | 2% |
| SEPICIDE™ HB : | | 1% |
| Eau : | q. s. p. | 100% |

### Exemple 75 : Gel crème teinté ultra naturel

### FORMULE

| | | | |
|---|---|---|---|
| A | eau : | | 10,0% |
| | Butylène glycol : | | 4,0% |
| | PEG-400: | | 4,0% |
| | PECOSIL™ PS100 : | | 1,5% |
| | NaOH : | q.s. | pH = 7 |
| | Dioxyde de titane : | | 2,0% |
| | Oxyde de fer jaune : | | 0,8% |
| | Oxyde de fer rouge : | | 0,3% |
| | Oxyde de fer noir : | | 0,05% |
| B | LANOL™ 99: | | 4,0% |
| | Caprylic capric triglycéride | | 4,0% |
| | SEPIFEEL™ ONE : | | 1,0% |
| | Composition 5 : | | 3,0% |
| C | eau: | q.s.p. | 100% |
| | MICROPEARL™ M305 : | | 2,0% |
| | EDTA tétrasodé : | | 0,05% |
| | DOW CORNING™ 245 Fluid : | | 4,0% |
| D | SEPICIDE™ HB : | | 0,5% |
| | SEPICIDE CI : | | 03% |
| | Parfum : | | 0,2% |

### MODE OPERATOIRE

préparer le mélange B + C puis ajouter A puis D.

### Exemple 76 : Soin pour les peaux grasses

### FORMULE

| | | | |
|---|---|---|---|
| A | MICROPEARL™ M310 : | | 1,0% |
| | Composition 5 : | | 5,0% |
| | Isononanoate d'octyle : | | 4.0% |
| B | eau : | q.s.p. | 100% |
| C | SEPICONTROL™ A5 : | | 4,0% |
| | Parfum : | | 0,1% |
| | SEPICIDE™ HB : | | 0,3% |
| | SEPICIDE™ CI : | | 0,2% |
| D | CAPIGEL™ 98 : | | 0,5% |
| | Eau : | | 10% |

### Exemple 77 : Crème aux AHA

### FORMULE

| | | | |
|---|---|---|---|
| A | MONTANOV™ 68 : | | 5,0% |
| | LIPACIDE™ PVB : | | 1,05% |
| | LANOL™ 99 : | | 10,0% |
| B | eau : | q.s.p. | 100% |
| | Acide gluconique : | | 1,5% |
| | TEA (triéthanolamine) : | | 0,9% |
| C | Composition 4 : | | 1,5% |
| D | parfum : | | 0,4% |
| | SEPICIDE™ HB : | | 0,2% |
| | SEPICIDE™ CI : | | 0,4% |

### Exemple 78 : Autobronzant non gras pour visage et corps

### FORMULE

| | | | |
|---|---|---|---|
| A | LANOL™ 2681 : | | 3,0% |
| | Composition 3 : | | 2,5% |
| B | eau : | q.s.p. | 100% |
| | Dihydroxyacétone : | | 3,0% |
| C | parfum : | | 0,2% |
| | SEPICIDE™ HB : | | 0,8% |
| | NaOH (hydroxyde de sodium) : | qs | pH = 5 |

### Exemple 79 : Lait solaire au monoï de Tahiti

### FORMULE

| | | | |
|---|---|---|---|
| A | Monoï de Tahiti : | | 10% |
| | LIPACIDE™ PVB : | | 0,5% |
| | Composition 7 : | | 2,2% |
| B | eau : | q.s.p. | 100% |
| C | parfum : | | 0,1% |
| | SEPICIDE™ HB : | | 0,3% |
| | SEPICIDE™ CI : | | 0,1% |
| | PARSOL™ MCX : | | 4,0% |

### Exemple 80 : Soin solaire pour le visage

### FORMULE

| | | | |
|---|---|---|---|
| A | DC™ 1501 : | | 4,0% |
| | Composition 5 : | | 3,5% |
| B | eau : | q.s.p. | 100% |
| C | parfum : | | 0,1% |
| | SEPICIDE™ HB : | | 0,3% |
| | SEPICIDE™ CI : | | 0,21% |
| | PARSOL™ MCX : | | 5,0% |
| | Micatitane : | | 2,0% |
| | Acide lactique : | q.s.p. | pH = 6,5 |

### Exemple 81 : Emulsion bronzante sans soleil

### FORMULE

| | | | |
|---|---|---|---|
| A | LANOL™ 99 : | | 15% |
| | MONTANOV™ 68 : | | 5,0% |
| | PARSOL™ MCX : | | 3,0% |
| B | eau : | q.s.p. | 100% |
| | Dihydroxyacétone : | | 5,0% |
| | Phosphate monosodique : | | 0,2% |
| C | Composition 1 : | | 0,5% |
| D | parfum : | | 0,3% |
| | SEPICIDE™ HB : | | 0,8% |
| | NaOH : | q.s. | pH=5. |

### Exemple 82 : Crème de soin

| | | |
|---|---|---|
| DOW CORNING™ 345 : | | 10% |
| Composition 8 : | | 0,8% |
| MONTANOV™ 68: | | 4,5% |
| Conservateur : | | 0,65% |
| Lysine : | | 0,025% |
| EDTA (sel disodique): | | 0,05% |
| KETROL™ T: | | 0,2% |
| Glycérine : | | 3,0% |
| Eau : | qsp | 100% |

### Exemple 83 : Crème de soin

| | | |
|---|---|---|
| DOW CORNING™ 345 : | | 10% |
| Composition 3 : | | 0,8% |
| MONTANOV™ 68: | | 4,5% |
| Perfluoropolyméthylisopropyléther : | | 0,5% |
| Conservateur : | | 0,65% |
| Lysine: | | 0,025% |
| EDTA (sel disodique): | | 0,05% |
| PEMULEN™ TR : | | 0,2% |
| Glycérine: | | 3% |
| Eau: | qsp | 100% |

### Exemple 84 : Lait corporel

### FORMULE

| | | | |
|---|---|---|---|
| A | SIMULSOL™ 165 : | | 5,0% |
| | LANOL™ 1688 : | | 12,0% |
| | LANOL™ 14 M : | | 2,0% |
| | Alcool cétylique : | | 0,3% |
| | SCHERCEMOL™ OP : | | 3% |
| B | Eau: | q.s.p. | 100% |
| C | Composition 4 : | | 0,35% |
| D | SEPICIDE™ CI : | | 0,2% |
| | SEPICIDE™ HB : | | 0,5% |
| | Parfum : | | 0,20% |

### MODE OPERATOIRE

Emulsionner B dans A vers 75°C ; ajouter C vers 60°C, puis D vers 30°C

### Exemple 85 : Gel soin de massage

### FORMULE

| | | | |
|---|---|---|---|
| A | Composition 5 : | | 3,00% |
| | Eau: | | 30% |
| B | SEPICIDE™ CI: | | 0,20% |
| | SEPICIDE™ HB : | | 0,30% |
| | Parfum : | | 0,05% |
| C | colorant: | | q.s. |
| | Eau: | q.s.p. | 100% |
| D | MICROPEARL™ SQL : | | 5,0% |
| | LANOL™ 1688 : | | 2% |

### MODE OPERATOIRE

Préparer A ; additionner B, puis C, puis D.

### Exemple 86 : Lait corporel

### FORMULE

| | | | |
|---|---|---|---|
| A | SEPIPERL™ N : | | 3,0% |
| | Triheptonate de glycérol : | | 10,0% |
| B | Eau : | q.s.p. | 100% |
| C | Composition 4 : | | 1,0% |
| D | Parfum : | | q.s. |
| | Conservateur : | | q.s. |

### MODE OPERATOIRE

Fondre A à environ 75°C. Emulsionner B dans A à 75°C ; ajouter C vers 60°C, puis D.

### Exemple 87 : Baume après-rasage apaisant sans alcool

| | | |
|---|---|---|
| Mélange de lauryl aminoacides : | | 0,1% à 5% |
| Aspartate de magnésium et de potassium : | | 0,002% à 0,5% |
| LANOL™ 99: | | 2% |
| Huile d'amandes douces : | | 0,5% |
| Eau: | q.s.p. | 100% |
| Composition 3 : | | 3% |
| SEPICIDE™ HB : | | 0,3% |
| SEPICIDE™ CI : | | 0,2% |
| Parfum : | | 0,4% |

### Exemple 88 : Lait corporel

| | | |
|---|---|---|
| SEPIPERL™ N : | | 3,5% |
| LANOL™ 37T : | | 8,0% |
| SOLAGUM™ L : | | 0,05% |
| Eau: | q.s.p. | 100% |
| Benzophénone 3 : | | 2,0% |
| Diméthicone 350 cPs : | | 0,05% |
| Composition 2 : | | 0,8% |
| Conservateur : | | 0,2% |
| Parfum : | | 0,4% |

### Exemple 89 : Baume après-rasage apaisant sans alcool

### FORMULE

| | | | |
|---|---|---|---|
| A | LIPACIDE™ PVB : | | 1,0% |
| | LANOL™ 99: | | 2,0% |
| | Huile d'amandes douces : | | 0,5% |
| B | Composition 1 : | | 3,5% |
| C | Eau: | q.s.p. | 100% |
| D | Parfum : | | 0,4% |
| | SEPICIDE™ HB : | | 0,4% |
| | SEPICIDE™ CI: | | 0,2% |

### Exemple 90: Gel rafraîchissant après-rasage

### FORMULE

| | | | |
|---|---|---|---|
| A | LIPACIDE™ PVB : | | 0,5% |
| | LANOL™ 99: | | 5,0% |
| | Composition 3 : | | 2,5% |
| B | eau: | q.s.p. | 100% |
| C | MICROPEARL™ LM : | | 0,5% |
| | Parfum : | | 0,2% |
| | SEPICIDE™ HB : | | 0,3% |
| | SEPICIDE™ CI : | | 0,2% |

### Exemple 91 : Crème aux AHA

### FORMULE

| | | | |
|---|---|---|---|
| A | MONTANOV™ 68: | | 5,0% |
| | LIPACIDE™ PVB : | | 1,05% |
| | LANOL™ 99: | | 10,0% |
| B | Eau: | q.s.p. | 100% |
| | Acide gluconique : | | 1,5% |
| | TEA (triéthanolamine) : | | 0,9% |
| C | Composition 3 : | | 1,5% |
| D | Parfum : | | 0,4% |
| | SEPICIDE™ HB : | | 0,2% |
| | SEPICIDE™ CI : | | 0,4% |

### Exemple 92 : Gel brillance

| | | |
|---|---|---|
| Composition 7 : | | 1,5% |
| Silicone volatile : | | 25% |
| Propylèneglycol : | | 25% |
| Eau déminéralisée: | | 10% |
| Glycérine : | qsp | 100% |

### Exemple 93 : Gel amincissant

| | | |
|---|---|---|
| Composition 6 : | | 1,5% |
| LANOL™ 99: | | 2% |
| Caféine : | | 5% |
| Ethanol : | | 40% |
| MICROPEARL™ LM : | | 2% |
| Eau déminéralisée: | qsp | 100% |
| Conservateur parfum : | | qs |

### Exemple 94 : Lait démaquillant

| | |
|---|---|
| SIMULSOL™ 165 : | 4% |
| MONTANOV™ 202 : | 1% |
| Caprylate-caprate triglyceride : | 15% |
| PECOSIL™ DCT : | 1% |
| Eau déminéralisée : | qs |
| CAPIGEL™ 98 : | 0,5% |
| Composition 4 | 1% |
| PROTEOL™ APL : | 2% |
| Hydroxyde de sodium : | qsp pH = 7 |

### Exemple 95 : Masque crème "rince off" restructurant pour cheveux stressés et fragilisés

| | |
|---|---|
| KETROL™ T : | 0,5% |
| PECOSIL™ SPP50 : | 0, 75% |
| N-cocoyl aminoacides : | 0,70% |
| Butylèneglycol: | 3,0% |
| Composition 1 : | 3,0% |
| MONTANOV™ 82 : | 3,0% |
| Huile de jojoba : | 1,0% |
| LANOL™ P : | 6,0% |
| AMONYL™ DM : | 1,0% |
| LANOL™ 99: | 5,0% |
| SEPICIDE™ HB : | 0,3% |
| SEPICIDE™CI: | 0,2% |
| Parfum : | 0,2% |
| Eau : | qsp 100% |

### Exemple 96 : Crème solaire

| | | |
|---|---|---|
| SIMULSOL™ 165 : | | 3% |
| MONTANOV™ 202 : | | 2% |
| Benzoate C12-C15 : | | 8% |
| PECOSIL™ PS 100 : | | 2% |
| Diméthicone: | | 2% |
| DOW CORNING™ 345 : | | 5% |
| PARSOL™ MCX: | | 6% |
| Benzophénone-3 : | | 4% |
| Oxyde de Titane : | | 8% |
| KETROL™ T : | | 0,2% |
| Butylèneglycol : | | 5% |
| Eau déminéralisée : | qsp | 100% |
| Composition 8 : | | 1,5% |
| Conservateur, parfum : | | qs |

### Exemple 97 : Gel de soin peaux mixtes

| | | |
|---|---|---|
| Composition 3 : | | 4% |
| Squalane végétal : | | 5% |
| Dimethicone : | | 1,5% |
| SEPICONTROL™ A5 : | | 4% |
| KETROL™ T: | | 0,3% |
| Eau: | qsp | 100% |
| Conservateur, Parfum : | | qs. |

### Exemple 98 : Lotion capillaire

| | | |
|---|---|---|
| Butylène glycol: | | 3,0% |
| Composition 4 : | | 3% |
| SIMULSOL™ 1293 : | | 3,0% |
| Acide lactique: | qs | pH = 6 |
| SEPICIDE™ HB : | | 0,2% |
| SEPICIDE™ CI : | | 0,3% |
| Parfum : | | 0,3% |
| Eau: | qs | 100% |

### Exemple 99 : Shampooing protecteur et relaxant

| | | |
|---|---|---|
| Amonyl™ 675 SB : | | 5,0% |
| Sodium lauryl éther sulfate à 28% : | | 35,0% |
| Composition 6 : | | 3,0% |
| SEPICIDE™ HB : | | 0,5% |
| SEPICIDE™ CI : | | 0,3% |
| Hydroxy de de sodium : | QS pH | = 7,2 |
| Parfum : | | 0,3% |
| Colorant (FDC bleu 1/jaune 5) : | | QS |
| Eau : | QSP | 100% |

### Exemple 100 : Protecteur "leave-on" ; Soin anti-stress pour cheveux

| | | |
|---|---|---|
| KETROL™ T: | | 0,5% |
| Mélange de cocoyl aminoacides: | | 3,0% |
| Butylèneglycol : | | 5,0% |
| DC 1501 : | | 5,0% |
| Composition 1 : | | 4,0% |
| SEPICIDE™ HB: | | 0,5% |
| SEPICIDE™CI: | | 0,3% |
| Parfum : | | 0,3% |
| Eau: | QSP | 100 |

### Exemple 101 : Crème vitaminée

| | | |
|---|---|---|
| SIMULSOL™ 165 : | | 5% |
| MONTANOV™ 202 : | | 1% |
| Caprylic/capric triglycérides : | | 20% |
| Palmitate de vitamine A : | | 0,2% |
| Acétate de vitamine E : | | 1% |
| MICROPEARL™ M 305 : | | 1,5% |
| Composition 2 : | | 2% |
| Eau | qsp | 100% |
| Conservateur, parfum | | qs |

### Exemple 102 : Gel solaire

### FORMULE

| | |
|---|---|
| Composition 5 : | 3,00% |
| SEPICIDE™ CI : | 0,20% |
| SEPICIDE™ HB : | 0,30% |
| Parfum : | 0,10% |
| Colorant : | qs |
| silice : | 3,00% |
| Eau: | q.s.p 100% |
| Huile de silicone : | 2,0% |
| Benzophénone - 3 : | 5,00% |

### Exemple 103 : Gloss lèvre

| | | |
|---|---|---|
| Composition 5 : | | 1,50% |
| Schercemol™ TISC | | 15,00% |
| Vistanol™ NPGC | | 15,00% |
| Candurin Paprika | | 0,50% |
| MONTANOX™ 80 | | 1,00% |
| Antaron™ V216 | | 0,90% |
| Arôme Abricot | | 0,20% |
| SEPICIDE™ HB | | 0,50% |
| C Maltidex™ H16322 | qsp | 100% |

### Exemple 104 : Poudre pressée Terre de soleil

| | | |
|---|---|---|
| Composition 3 : | | 2,00% |
| LANOL™ 99 | | 12,00% |
| SEPIWHITE™ MSH | | 1,00% |
| Talc | | 33,00% |
| MICROPEARL™ M310 | | 3,00% |
| Oxyde de fer jaune | | 0,80% |
| Oxyde de fer rouge | | 0,30% |
| Oxyde de fer noir | | 0,05% |
| Mica : | qs | 100% |

### Exemple 105 : Emulsion pour peaux à tendance atopique

| | |
|---|---|
| Arlacel™ P135 : | 2,00% |
| Composition 6 : | 1,00% |
| Lanol™ 1688 : | 14,00% |
| Primol™ 352 | 8,00% |
| Glycérine | 5,00% |
| Eau | qsp 100% |
| Sulfate de magnésium | 0,70% |
| SEPICIDE™ HB | 0,30% |
| SEPICIDE™ CI | 0,20% |
| MICROPEARL™ M310 | 5,00% |

### Exemple 106 : Soin solaire apaisant (eau dans silicone)

| | |
|---|---|
| Composition 8 : | 2,00% |
| DC5225C : | 20,00% |
| DC345 | 10,00% |
| SEPICALM™ VG | 3,00% |
| Dioxide de titane MT100T | 5,00% |
| Oxyde de zinc Z cote HP1 | 5,00% |
| SEPICIDE™ HB | 0,30% |
| Parfum | 0,05% |
| SEPICIDE™ CI | 0,20% |
| Glycérine | 5,00% |
| Chlorure de sodium | 2,00% |
| Eau | qsp 100% |

### Exemple 107 : Soin multi-phases

| | |
|---|---|
| Composition 7 | 3,00% |
| C12-15 alkylbenzoate | 25,00% |
| AQUAXYL™ | 3,00% |
| SEPITONIC™ M3 | 1,00% |
| SEPICIDE™ HB | 0,50% |
| SEPICIDE™ CI | 0,30% |
| Eau | qsp 100% |

Les définitions des produits commerciaux utilisés dans les exemples sont les suivantes :
SIMULSOL™ 1293 est de l'huile de castor hydrogénée et éthoxylée, avec un indice d'éthoxylation égal à 40, commercialisé par la société SEPPIC.
CAPIGEL™ 98 est un épaississant liquide à base de copolymère acrylate commercialisé pas la société SEPPIC.
KETROL™ T est de la gomme de xanthane commercialisée par la société KELCO.
LANOL™ 99 est de l'isononanoate d'isononyle commercialisé par la société SEPPIC.
DC1501 est un mélange de cyclopentasiloxane et de diméthiconol commercialisé par la société DOW CHEMICAL.
MONTANOV™ 82 est un agent émulsionnant à base d'alcool cétéarylique et de cocoylglucoside.
Le MONTANOV™ 68 (cétéaryl glucoside), est une composition auto-émulsionnable telle que décrite dans WO 92/06778, commercialisée par la société SEPPIC.
Le MICROPEARL™ M100 est une poudre ultra fine au toucher très doux et à action matifiante commercialisée par la société MATSUMO
Le SEPICIDE™ CI, imidazolidine urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL^{®} S sont des facteurs de consistance commercialisés par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
L'AQUAXYL™ est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
PARSOL™ MCX est du paraméthoxycinnamate d'octyle commercialisé par la société GI-VAUDAN.
Le SEPIPERL™ N est un agent nacrant, commercialisé par la société SEPPIC, à base d'un mélange d'alkyl poly glucosides tels que ceux décrits dans WO 95/13863.
Le MICROPEARL™ SQL est un mélange de micro particules renfermant du squalane qui se libère sous l'action du massage; il est commercialisé par la société MATSUMO.
Le LANOL™ 37T est du triheptanoate de glycérol, commercialisé par la société SEPPIC.
Le SOLAGUM™ L est un carraghénane commercialisé par la société SEPPIC.
Le MARCOL™ 82 est une huile de paraffine commercialisée par la société EXXON.
Le LANOL™ 84D est du malate de dioctyle commercialisé par la société SEPPIC.
Le PARSOL NOX™ est un filtre solaire commercialisé par la société GIVAUDAN.
l'EUSOLEX™ 4360 est un filtre solaire commercialisé par la société MERCK.
Le DOW CORNING™ 245 Fluid est de la cyclométhicone, commercialisée par la société DOW CORNING.
Le LIPACIDE™ PVB, est un hydrolysat de protéines de blé acylé commercialisé par la société SEPPIC.
Le MICROPEARL™ LM est un mélange de squalane, de polyméthylméthacrylate et de menthol, commercialisé par la société SEPPIC.
Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
Le LANOL™ 2681 est un mélange caprylate, caprate de coprah, commercialisé par la société SEPPIC.
Le MONTANOV™ 202, est une composition APG/alcools gras telle que décrite dans WO9 98/47610, commercialisée par la société SEPPIC.
Le PROTEOL™ APL est un tensioactif moussant, commercialisée par la société SEPPIC
Le SCHERCEMOL™ TISC est un ester (citrate de tri-isostéaryle) commercialisé par la société SCHER.
Le VISTANOL™ NPGC est un ester (néopentyl glycol dicaprate) commercialisé par la société SEWA KASEI.
L'ANTARON™ V216 est un polymère synthétique (PVP/hexadecene copolymer) distribué par la société UNIVAR.
Le C MALTIDEX™ H16322 est polyol (sirop de maltitol) commercialisé par la société CERESTAR.
Le SEPIWHITE™ MSH est un actif dépigmentant (undecylenoyl phenylalanine) commercialisé par la société SEPPIC.
Le DC 345 est un cyclométhicone commercialisé par la société Dow Corning.
Le DC 5225C est un mélange de cyclopentasiloxane et de diméthicone copolyol commercialisé par la société DOW CORNING.
Le SEPICALM™ VG est un actif apaisant (sodium palmitoylproline) commercialisé par la société SEPPIC.
Le MT100VT est un dioxyde de titane micronisé ayant subi un traitement de surface (hydroxyde d'aluminium / acide stéarique) distribué par la société UNIPEX.
Le Z COTE HP1 est un oxyde de zinc micronisé ayant subi un traitement de surface distribué par GATTEFOSSE.
Le CANDURIN PAPRIKA est un mélange de silicate de potassium et d'aluminium et d'oxyde de fer.
Le MONTEINE™ CA est un agent hydratant commercialisé par la société SEPPIC.

## Revendications

1. Composition sous forme d'un latex inverse comprenant :
a) de 50 % en poids à 80 % d'au moins un polymère organique (P) linéaire, branché ou réticulé,
b) de 5 % en poids à 10 % d'un système émulsionnant (S₁) de type eau dans huile (E/H),
c) de 5 % en poids à 45 % en poids d'au moins une huile, et
d) de 0 % à 5 % d'eau,
et **caractérisée en ce que** de 0,01 % à 10 % en proportions molaires des motifs monomériques que ledit polymère P comporte, est un monomère neutre de formule (I'):
CH₂=CH-C(=O)-O-(CH₂-CH₂-O)ₙ-R'₄ (I')
dans laquelle le radical R'₄ représente un radical aliphatique choisi parmi les radicaux, octyle, decyle, dodecyle, tétradécyle, hexadécyle, octadécyle, eicosyle, docosyle, tétracosyle et n' représente un nombre compris entre 4 et 25 ; ou un monomère de formule (I") :
CH₂=C(CH₃)-C(=O)-O-(CH₂-CH₂-O)n" -R"₄ (I")
dans laquelle le radical R"₄ représente un radical aliphatique choisi parmi les radicaux octyle, dodécyle, tétradécyle, hexadécyle, octadécyle, eicosyle, docosyle, tétracosyle et n" représente un nombre compris entre 4 et 25.

2. Composition telle que définie à la revendication 1, dans laquelle de 0,05% à 5% en proportions molaires, et de préférence de 0,1% à 1% en proportions molaires, des motifs monomériques que ledit polymère P comporte au moins un monomère neutre de formule (I).

3. Composition telle que définie à l'une des revendications 1 ou 2, dans laquelle le polymère (P) est un copolymère dans lequel chacun des monomères différent du composé de formule (I') ou de formule (I"), est choisi indépendamment l'un de l'autre ou bien parmi ceux possédant une fonction acide fort partiellement ou totalement salifiée ou bien parmi ceux possédant une fonction acide faible partiellement ou totalement salifié ou bien parmi les monomères neutres.

4. Composition telle que définie à l'une des revendications 1 à 3, dans laquelle le polymère (P) est réticulé avec un composé diéthylénique ou polyéthylènique dans la pro-portion molaire exprimée par rapport aux monomères mis en oeuvre, inférieure ou égale à 0,25%, et plus particulièrement inférieure ou égale à 0,05% et tout particulièrement entre 0,005% et 0,01%.

5. Composition telle que définie à la revendication 4, pour laquelle l'agent de réticulation et/ou l'agent de ramification est choisi parmi le diméthacrylate d'éthylèneglycol, le diacrylate de diéthylèneglycol, le diallyloxyacétate de sodium, le diacrylate d'éthylèneglycol, le diallyl urée, le triallylamine, le triméthylol propanetriacrylate, le méthylène-bis-(acrylamide) ou un mélange de ces composés.

6. Composition telle que définie à l'une des revendications 1 à 5, pour laquelle le monomère à fonction acide fort, que le polymère (P) comporte est l'acide 2-méthyl 2-[(1- oxo 2-propènyl) amino] 1-propanesulfonique partiellement ou totalement salifié.

7. Composition telle que définie à l'une des revendications 1 à 6, pour laquelle les monomères à fonction acide faible, que le polymère (P) comportent sont choisis parmi l'acide acrylique, l'acide méthacrylique, l'acide itaconique, l'acide maléique ou l'acide 3-méthyl 3-[(1-oxo 2-propènyl) amino] butanoïque partiellement ou totalement salifié.

8. Composition telle que définie à l'une des revendications 1 à 7, pour laquelle les monomères neutres différents du composé de formule (I) que le polymère (P) comportent sont choisis parmi l'acrylamide, le méthacrylamide, le diacétoneacrylamide, le diméthylacrylamide, le N-isopropyl acrylamide, le N-[2-hydroxy 1,1-bis(hydroxyméthyl) éthyl] propénamide, l'acrylate de (2-hydroxy éthyle), l'acrylate de (2,3-dihydroxy propyle), le méthacrylate de (2-hydroxy éthyle), le méthacrylate de (2,3-dihydroxy propyle), un dérivé éthoxylé de poids moléculaire compris entre 400 et 1000, de chacun de ces esters ou le vinyl pyrrolidone.

9. Composition telle que définie à l'une des revendications 1 à 8, dans laquelle le polymère (P) est choisi parmi :
- les copolymères réticulés de l'acide acrylique partiellement salifié sous forme de sel de sodium ou de sel d'ammonium, de l'acrylamide et de l'acrylate de lauryle tétraéthoxylé ;
- les copolymères réticulés de l'acide 2-méthyl 2-[(1-oxo 2-propènyl) amino] 1-propanesulfonique partiellement salifié sous forme de sel de sodium de l'acrylamide et de l'acrylate de lauryle tétraéthoxylé ;
- les copolymères réticulés de l'acide 2-méthyl 2-[(1-oxo 2-propènyl) amino] 1-propanesulfonique de l'acide acrylique partiellement salifiés sous forme de sel de sodium et de l'acrylate de lauryle tétraéthoxylé ;
- les copolymères réticulés de l'acide 2-méthyl 2-[(1-oxo 2-propènyl) amino] 1-propanesulfonique partiellement salifié sous forme de sel de sodium de l'acrylate de 2-hydroxy éthyle et de l'acrylate de lauryle tétraéthoxylé ;
- les copolymères réticulés de l'acide 2-méthyl 2-[(1-oxo 2-propènyl) amino] 1-propanesulfonique partiellement salifié sous forme de sel de sodium et de l'acrylate de lauryle tétraéthoxylé ;
- les copolymères réticulés de l'acide acrylique partiellement salifié sous forme de sel d'ammonium ou de sel de monoéthanolamine et de l'acrylate de lauryle tétraéthoxylé ;
- les copolymères réticulés de l'acrylamide, de l'acide 2-méthyl 2-[(1-oxo 2-propènyl) amino] 1-propanesulfonique de l'acide acrylique partiellement salifiés sous forme de sel sodium et de l'acrylate de lauryle tétraéthoxylé ; et
- les copolymères de l'acide 2-méthyl 2-[(1-oxo 2-propènyl) amino] 1-propanesulfonique partiellement salifié sous forme de sel de sodium, de l'acrylamide, du vinyl pyrrolidone et de l'acrylate de lauryle tétraéthoxylé.

10. Composition telle que définie à l'une des revendications 1 à 9, comprenant de 60% en poids à 70% en poids de polymère (P).

11. Composition telle que définie à l'une des revendications 1 à 10, comprenant en outre jusqu'à 5% de son poids d'un système émulsionnant (S₂) de type huile dans eau (H/E).

12. Procédé de préparation de la composition telle que à l'une des revendications 1 à 11, **caractérisé en ce que** :
a) l'on émulsionne une phase aqueuse (A) contenant les monomères et les éventuels additifs hydrophiles, dans une phase organique (O) contenant, le système tensioactif (S₁), un mélange constitué de l'huile destinée à être présente dans la composition finale et d'une huile volatile et les éventuels additifs hydrophobes,
b) l'on amorce la réaction de polymérisation par introduction dans l'émulsion formée en a), d'un initiateur de radicaux libres puis on la laisse se dérouler, et
c) l'on concentre par distillation, le milieu réactionnel issu de l'étape b), jusqu'à élimination complète de ladite huile volatile.

13. Procédé tel que défini à la revendication 12, dans lequel à l'issu de l'étape c), on introduit un ou plusieurs agents émulsifiants de type huile dans eau à une température inférieure à 50°C.

14. Utilisation de la composition telle que définie à l'une des revendications 1 à 11, comme épaississant et/ou émulsionnant de composition topique, cosmétique, dermopharmaceutique ou pharmaceutique.

## Patentansprüche

1. Zusammensetzung in Form eines inversen Latex, enthaltend:
a) 50 Gew.-% bis 80 Gew.% mindestens eines linearen, verzweigten oder vernetzten organischen Polymers (P),
b) 5 Gew.-% bis 10 Gew.-% eines Emulgatorsystems (S₁) des Wassser-in-Öl-Typs (W/O),
c) 5 Gew.-% bis 45 Gew.-% mindestens eines Öls und
d) 0 % bis 5 % Wasser,
und **dadurch gekennzeichnet, dass** 0,01 % bis 10 % in Molverhältnissen der Monomereinheiten, die das Polymer P aufweist, ein neutrales Monomer der Formel (I') ist:
CH₂=CH-C(=O)-O-(CH₂₋CH₂₋O)_{n'}-R'₄ (I')
worin das Radikal R'₄ ein aliphatisches Radikal darstellt, das ausgewählt ist aus den Octyl-, Decyl-, Dodecyl-, Tetradecyl-, Hexadecyl-, Octadecyl-, Eicosyl, Docosyl-, Tetracosyl-Radikalen, und n' eine Zahl zwischen 4 und 25 darstellt;
oder ein Monomer der Formel (I") ist:
CH₂=C(CH₃)-C(=O)-O-(CH₂-CH₂-O)ₙ" -R"₄ (I")
worin das Radikal R"₄ ein aliphatisches Radikal darstellt, das ausgewählt ist aus den Octyl-, Dodecyl-, Tetradecyl-, Hexadecyl-, Octadecyl-, Eicosyl, Docosyl-, Tetracosyl-Radikalen, und n" eine Zahl zwischen 4 und 25 darstellt.

2. Zusammensetzung nach Anspruch 1, in der 0,05 % bis 5 %, in Molverhältnissen, bevorzugt 0,1 % bis 1 % in Molverhältnissen der Monomereinheiten, die das Polymer P aufweist, ein neutrales Monomer der Formel (I) ist.

3. Zusammensetzung nach einem der Ansprüche 1 oder 2, in der das Polymer (P) ein Copolymer ist, in dem jedes der von der Verbindung der Formel (I') oder der Formel (I") verschiedenen Monomere unabhängig voneinander oder aus jenen ausgewählt ist, die eine Säurefunktion haben, die stark teilweise oder vollständig in ein Salz überführt ist, oder aus jenen ausgewählt ist, die eine Säurefunktion haben, die schwach teilweise oder vollständig in ein Salz überführt ist, oder aus den neutralen Monomeren ausgewählt ist.

4. Zusammensetzung nach einem der Ansprüche 1 bis 3, in der das Polymer (P) mit einer Diethylenverbindung oder Polyethylenverbindung in dem Molverhältnis vernetzt ist, das in Bezug auf die verwendeten Monomere ausgedrückt weniger oder gleich 0,25 % und insbesondere weniger oder gleich 0,05 % und ganz besonders zwischen 0,005 % und 0,01 % beträgt.

5. Zusammensetzung nach Anspruch 4, bei der das Vernetzungsmittel und/oder das Verzweigungsmittel ausgewählt ist aus Ethylenglycoldimethacrylat, Diethylenglycoldiacrylat, Natrium-Diallyloxyacetat, Ethylenglycoldiacrylat, Diallylurea, Triallylamin, Trimethylolpropantriacrylat, Methylenbisacrylamid oder einem Gemisch aus diesen Verbindungen.

6. Zusammensetzung nach einem der Ansprüche 1 bis 5, bei der das Monomer mit starker Säurefunktion, welches das Polymer (P) aufweist, 2-Methyl-2-[(1-oxo-2-propenyl)amino]-1-propansulfonsäure ist, die teilweise oder vollständig in ein Salz überführt ist.

7. Zusammensetzung nach einem der Ansprüche 1 bis 6, bei der die Monomere mit schwacher Säurefunktion, die das Polymer (P) aufweisen, ausgewählt sind aus Acrylsäure, Methacrylsäure, Itakonsäure, Maleinsäure oder 3-Methyl-3-[(1-oxo-2-propenyl)amino]butansäure, die teilweise oder vollständig in ein Salz überführt ist.

8. Zusammensetzung nach einem der Ansprüche 1 bis 7, bei der die von der Verbindung der Formel (I) verschiedenen neutralen Monomere, die das Polymer (P) aufweisen, ausgewählt sind aus Acrylamid, Methacrylamid, Diacetonacrylamid, Dimethylacrylamid, N-Isopropylacrylamid, N-[2-Hydroxy-1,1-bis(Hydroxymethyl)ethyl]propenamid, (2-Hydroxyethyl)acrylat, (2,3-Dihydroxypropyl)acrylat, (2-Hydroxyethyl)methacrylat, (2,3-Dihydroxypropyl)methacrylat, einem Ethoxyl-Derivat mit einem Molgewicht zwischen 400 und 1 000 jedem dieser Ester oder Vinylpyrrolidon.

9. Zusammensetzung nach einem der Ansprüche 1 bis 8, in der das Polymer (P) ausgewählt ist aus:
- den vernetzten Copolymeren der Acrylsäure, die teilweise in ein Salz überführt ist in Form von Natrium- oder Ammoniumsalz, von Acrylamid und von Tetraethoxyllaurylacrylat;
- den vernetzten Copolymeren der 2-Methyl-2-[(1-oxo-2-propenyl)amino]-1-propansulfonsäure, die teilweise in ein Salz überführt ist in Form von Natrium-Acrylamid-Salz und von Tetraethoxyllaurylacrylat;
- den vernetzten Copolymeren der 2-Methyl-2-[(1-oxo-2-propenyl)amino]-1-propansulfonsäure der Acrylsäure, die teilweise in ein Salz überführt sind in Form von Natriumsalz und von Tetraethoxyllaurylacrylat;
- den vernetzten Copolymeren der 2-Methyl-2-[(1-oxo-2-propenyl)amino]-1-propansulfonsäure, die teilweise in ein Salz überführt ist in Form von Natriumsalz von (2-Hydroxyethyl)acrylat und von Tetraethoxyllaurylacrylat;
- den vernetzten Copolymeren der 2-Methyl-2-[(1-oxo-2-propenyl)amino]-1-propansulfonsäure, die teilweise in ein Salz überführt ist in Form von Natriumsalz und von Tetraethoxyllaurylacrylat;
- den vernetzten Copolymeren der Acrylsäure, die teilweise in ein Salz überführt ist in Form von Ammoniumsalz oder von Monoethanolaminsalz und von Tetraethoxyllaurylacrylat;
- den vernetzten Copolymeren von Acrylamid, 2-Methyl-2-[(1-oxo-2-propenyl)amino]-1-propansulfonsäure der Acrylsäure, die teilweise in ein Salz überführt ist in Form von Natriumsalz und von Tetraethoxyllaurylacrylat; und
- den vernetzten Copolymeren der 2-Methyl-2-[(1-oxo-2-propenyl)amino]-1-propansulfonsäure, die teilweise in ein Salz überführt ist in Form von Natriumsalz, von Acrylamid, von Vinylpyrrolidon und von Tetraethoxyllaurylacrylat.

10. Zusammensetzung nach einem der Ansprüche 1 bis 9, enthaltend 60 Gew.-% bis 70 Gew.-% Polymer (P).

11. Zusammensetzung nach einem der Ansprüche 1 bis 10, enthaltend ferner bis zu 5 % ihres Gewichts eines Emulgatorsystems (S₂) des Typs Öl-in-Wasser (O/W).

12. Verfahren zur Herstellung der Zusammensetzung nach einem der Ansprüche 1 bis 11, **dadurch gekennzeichnet, dass**
a) man eine wässrige Phase (A), welche die Monomere und die eventuellen hydrophilen Zusatzstoffe enthält, in einer organischen Phase (O) emulgiert, die das Tensidsystem (S₁), ein Gemisch, das aus dem Öl, das dazu bestimmt ist, in der endgültigen Zusammensetzung vorhanden zu sein, und einem flüchtigen Öl besteht, und die eventuellen hydrophoben Zusatzstoffe enthält,
b) man die Polymerisationsreaktion durch Einbringen eines Initiators freier Radikaler in die in a) gebildete Emulsion einleitet und man sie anschließend sich entwickeln lässt und
c) man das aus Schritt b) hervorgegangene Reaktionsmedium durch Destillation bis zum vollständigen Entfernen des flüchtigen Öls konzentriert.

13. Verfahren nach Anspruch 12, bei dem man am Ende von Schritt c) einen oder mehrere Emulgatoren des Öl-in-Wasser-Typs bei einer Temperatur von unter 50 °C einbringt.

14. Verwendung der Zusammensetzung nach einem der Ansprüche 1 bis 11 als Verdickungsmittel und/oder Emulgator einer topischen, kosmetischen, dermopharmazeutischen oder pharmazeutischen Zusammensetzung.

## Claims

1. Composition in the form of an inverse latex comprising:
a) from 50 % by weight to 80 % of at least one linear, branched or crosslinked organic polymer (P),
b) from 5 % by weight to 10 % of a water-in-oil (W/O) type emulsifying system (S₁),
c) from 5 % by weight to 45 % by weight of at least one oil, and
d) from 0 % to 5 % of water,
and **characterised in that** from 0.01 % to 10 % in molar proportions of the monomer units that said polymer P comprises is at least one neutral monomer of formula (I'):
CH₂=CH-C(=O)-O-(CH₂-CH₂-O)ₙ-R'₄ (I')
the radical R'₄ representing an aliphatic radical selected from among the radicals octyl, decyl, dodecyl, tetradecyl, hexadecyl, octadecyl, eicosyl, docosyl, tetracosyl, and n' represents a number between 4 and 25; or a monomer of formula (I"):
CH₂=C(CH₃)-C(=O)-O-(CH₂-CH₂-O)n"-R"₄ (I")
the radical R"4 representing an aliphatic radical selected from among the radicals octyl, dodecyl, tetradecyl, hexadecyl, octadecyl, eicosyl, docosyl, tetracosyl and n" represents a number between 4 and 25.

2. Composition according to claim 1, wherein from 0.05 mol.% to 5 mol.% and preferably from 0.1 mol.% to 1 mol.% of the monomer units that said polymer P comprises is at least one neutral monomer of formula (I).

3. Composition according to either claim 1 or claim 2, wherein the polymer (P) is a copolymer in which each of the various monomers of the compound of formula (I') or of formula (I") is selected independently of the others either from those having a partially or completely salified strong acid function or from those having a partially or completely salified weak acid function or from the neutral monomers.

4. Composition according to any of claims 1 to 3, wherein the polymer (P) is crosslinked with a diethylenic or polyethylenic compound in a molar proportion, expressed relative to the monomers used, of less than or equal to 0.25 %, more particularly less than or equal to 0.05 % and most particularly between 0.005 % and 0.01 %.

5. Composition according to claim 4, for which the crosslinking agent and/or the branching agent is selected from ethylene glycol dimethacrylate, diethylene glycol diacrylate, sodium diallyloxyacetate, ethylene glycol diacrylate, diallyl urea, triallylamine, trimethylolpropane triacrylate, methylenebis(acrylamide), or a mixture of these compounds.

6. Composition according to any of claims 1 to 5, for which the monomer containing a strong acid function, which the polymer (P) comprises, is partially or completely salified 2-methyl-2-[(1-oxo-2-propenyl)amino]-1-propanesulfonic acid.

7. Composition according to any of claims 1 to 6, for which the monomers having a weak acid function, which the polymer (P) comprises, are selected from acrylic acid, methacrylic acid, itaconic acid, maleic acid or partially or completely salified 3-methyl-3-[(1-oxo-2-propenyl)amino]butanoic acid.

8. Composition according to any of claims 1 to 7, for which the neutral monomers other than the compound of formula (I), which the polymer (P) comprises, are selected from acrylamide, methacrylamide, diacetoneacrylamide, dimethylacrylamide, N-isopropyl-acrylamide, N-[2-hydroxy-1,1-bis(hydroxymethyl)ethyl]-propenamide, (2-hydroxyethyl)acrylate, (2,3-dihydroxypropyl)acrylate, (2-hydroxyethyl)methacrylate, (2,3-dihydroxypropyl)methacrylate, and an ethoxylated derivative having a molecular weight of between 400 and 1000, of each of these esters, or vinylpyrrolidone.

9. Composition according to any of claims 1 to 8, wherein the polymer (P) is selected from:
- crosslinked copolymers of acrylic acid partially salified in the form of a sodium salt or of an ammonium salt, of acrylamide and of tetraethoxylated lauryl acrylate;
- crosslinked copolymers of 2-methyl-2-[(1-oxo-2-propenyl)amino]-1-propanesulfonic acid partially salified in the form of a sodium salt, of acrylamide and of tetraethoxylated lauryl acryate;
- crosslinked copolymers of 2-methyl-2-[(1-oxo-2-propenyl)amino]-1-propanesulfonic acid and of acrylic acid, which are partially salified in the form of a sodium salt, and of tetraethoxylated lauryl acrylate;
- crosslinked copolymers of 2-methyl-2-[(1-oxo-2-propenyl)amino]-1-propanesulfonic acid partially salified in the form of a sodium salt, of 2-hydroxyethyl acrylate and of tetraethoxylated lauryl acrylate;
- crosslinked copolymers of 2-methyl-2-[(1-oxo-2-propenyl)amino]-1-propanesulfonic acid partially salified in the form of a sodium salt, and of tetraethoxylated lauryl acrylate;
- crosslinked copolymers of acrylic acid partially salified in the form of an ammonium salt or of a monoethanolamine salt, and of tetraethoxylated lauryl acrylate;
- crosslinked copolymers of acrylamide, of 2-methyl-2-[(1-oxo-2-propenyl)amino]-1-propanesulfonic acid and of acrylic acid, which are partially salified in the form of a sodium salt, and of tetraethoxylated lauryl acrylate; and
- copolymers of 2-methyl-2-[(1-oxo-2-propenyl)-amino]-1-propanesulfonic acid partially salified in the form of a sodium salt, of acrylamide, of vinylpyrrolidone and of tetraethoxylated lauryl acrylate.

10. Composition according to any of claims 1 to 9, comprising from 60 % by weight to 70 % by weight of the polymer (P).

11. Composition according to any of claims 1 to 10, also comprising up to 5 % of its weight of an oil-in-water (O/W) type emulsifying system (S₂).

12. Process for preparing the composition according to any of claims 1 to 11, **characterised in that**:
a) an aqueous phase (A) containing the monomers and the optional hydrophilic additives is emulsified in an organic phase (O) containing the surfactant system (S₁), a mixture consisting of the oil intended to be present in the final composition and of a volatile oil and the optional hydrophobic additives,
b) the polymerisation reaction is initiated by introduction into the emulsion formed in a) of a free-radical initiator, and the reaction is then left to proceed,
and
c) the reaction medium obtained from step b) is concentrated by distillation until said volatile oil has been completely removed.

13. Process according to claim 12, wherein, after step c), one or more oil-in-water type emulsifying agents are introduced at a temperature below 50 °C.

14. Use of the composition according to any of claims 1 to 11 as a thickener and/or emulsifier of a topical, cosmetic, dermopharmaceutical or pharmaceutical composition.
